(19) Europäisches Patentamt
European Patent Office
Office européen des brevets

(11) EP 4 726 043 A2

(12) EUROPEAN PATENT APPLICATION

(43) Date of publication:
**15.04.2026 Bulletin 2026/16**

(21) Application number: **26161439.0**

(22) Date of filing: **18.02.2020**

(51) International Patent Classification (IPC):
***C12N 15/113*** *(2010.01)*

(52) Cooperative Patent Classification (CPC):
**C12N 15/1031; C12P 19/34;** C12N 15/70

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.02.2019 JP 2019026868**
**25.09.2019 JP 2019174557**

(62) Document number(s) of the earlier application(s) in accordance with Art. 76 EPC:
**20759682.6 / 3 929 205**

(71) Applicant: **AJINOMOTO CO., INC.**
**Chuo-ku**
**Tokyo 104-8315 (JP)**

(72) Inventors:
- **TAKAHASHI, Daisuke**
  **Kanagawa, 210-8681 (JP)**
- **HAGIWARA, Yusuke**
  **Kanagawa, 210-8681 (JP)**
- **KAJIMOTO, Shohei**
  **Kanagawa, 210-8681 (JP)**
- **KONISHI, Miwa**
  **Kanagawa, 210-8681 (JP)**

(74) Representative: **Mewburn Ellis LLP**
**Aurora Building**
**Counterslip**
**Bristol BS1 6BX (GB)**

Remarks:
•The complete document including Reference Table(s) and the Sequence Listing(s) can be downloaded from the EPO website
•This application was filed on 27.02.2026 as a divisional application to the application mentioned under INID code 62.

# (54) METHOD FOR PRODUCING MODIFIED OLIGONUCLEOTIDE INCLUDING COMPLEMENTARY SEQUENCE

(57) The present invention provides a method for efficiently producing an oligonucleotide comprising a complementary portion, such as an siRNA and a heteroduplex oligonucleotide. More specifically, provided is a method for producing a modified oligonucleotide comprising a complementary portion having 11 to 27 nucleotide length, in which the method includes formation of the modified oligonucleotide by treating four or more oligonucleotide raw material fragments in total in the presence of an oligonucleotide ligase; the four or more oligonucleotide raw material fragments in total correspond to oligonucleotide raw material fragments that are obtained by dividing the modified oligonucleotide at a fragment linking site that satisfies following conditions (i) to (v): (i) one or more fragment linking sites are present in the complementary portion in each strand side, and two or more fragment linking sites in total are present in the modified oligonucleotide; (ii) when the modified oligonucleotide is divided at the fragment linking site, a sticky end is formed in the complementary portion, in which the sticky end has 1 to 10 nucleotide length; (iii) at least one oligonucleotide raw material fragment has a modified nucleotide; (iv) four oligonucleotide raw material fragments out of the four or more oligonucleotide raw material fragments in total include the complementary portion having 5 to 25 nucleotide length; and (v) total nucleotide length of the oligonucleotide raw material fragments corresponding to the complementary portions in each strand side is 11 to 27.

EP 4 726 043 A2

## Description

TECHNICAL FIELD

The present invention relates to a method for producing a modified oligonucleotide comprising a complementary portion.

BACKGROUND

Since usefulness of oligonucleotides such as an siRNA and an antisense in a nucleic acid drug is indicated, development thereof is actively carried out in recent years. Oligonucleotides are produced mainly by a synthetic method. For example, they can be produced by a solid phase synthesis such as a phosphoramidite method in which a nucleotide residue is linearly extended by one nucleotide at a time in succession. However, this method has problems, among other things, that purity and yield of the product decrease as the length of the oligonucleotide strand becomes longer, and that production efficiency is low. Therefore, a parallel synthetic method is wanted in which short oligonucleotide fragments are first synthesized, then followed by condensation thereof to obtain a target oligonucleotide.

Patent Literature 1 discloses the method of producing a single strand oligonucleotide by annealing a plurality of oligonucleotide raw material fragments corresponding to fragments of a target oligonucleotide with a template oligonucleotide that is complementary to the target oligonucleotide, enzymatically condensing the annealed oligonucleotide raw material fragments with each other, and separating the resulting target oligonucleotide strand from the template oligonucleotide.

Non-Patent Literature 1 describes PEGylation of an oligo-DNA by linking an oligo-DNA fragment and a PEGylated oligo-DNA fragment with a DNA ligase at the cohesive ends thereof. However, in Non-Patent Literature 1, only a natural type oligo-DNA fragment is used; thus, it is not clear whether the oligonucleotide comprising a complementary portion can be enzymatically condensed when using the oligonucleotide raw material comprising a short and modified type nucleotide because a low annealing property is suggested in the oligonucleotide like this.

Non-Patent Literatures 2 and 3 describe linking a nick formed by two oligonucleotide raw material fragments that are annealed with single strand oligonucleotide complementary thereto by using a ligase.

Non-Patent Literature 4 describes forming a double strand RNA of 48mer or larger by linking 24mer double strand oligo RNAs having a cohesive end by using an RNA ligase. However, in Non-Patent Literature 4, the lengths of both the substrate and product are long since the lengths of the substrate and product are 24 and 48 nucleotides, respectively. Consequently, the synthesis is carried out only under the condition of a high annealing ability of the substrate. Therefore, it is still unknown whether an enzymatic reaction takes place when an oligonucleotide substrate having a short strand and introduction of modified type nucleotides is used to condense two or more condensation sites, because the oligonucleotide substrate is expected to be low in the linking activity of the enzyme due to the short strand and introduction that are suggested to significantly lower the annealing ability.

Non-Patent Literature 5 describes that an siRNA was synthetically prepared.

Non-Patent Literature 6 discloses that the RNA ligase DraRnl belongs to the Rnl5 family.

PRIOR ART LITERATURES

PATENT LITERATURES

Patent Literature 1: US Patent Application Laid-open No. 2018/0023122

NON-PATENT LITERATURE

Non-Patent Literature 1: Sosic A, Pasqualin M, Pasut G, Gatto B. 2014. Enzymatic formation of PEGylated oligonucleotides. Bioconjug Chem 25: 433-441.

Non-Patent Literature 2: Bullard, D. R., & Bowater R. P. (2006). Direct comparison of nick-joining activity of the nucleic acid ligases from bacteriophage T4. Biochem. J, 398, 135-144.

Non-Patent Literature 3: Nandakumar, J., & Shuman, S. (2004). How an RNA Ligase Discriminates RNA versus DNA Damage. Molecular Cell, 16(2), 211-221.

Non-Patent Literature 4: Nandakumar, J, Ho CK, Lima CD, Shuman S. 2004. RNA substrate specificity and structure-guided mutational analysis of bacteriophage T4 RNA ligase 2. J Biol Chem 279: 31337-31347.

Non-Patent Literature 5: Jayaprakash K. Nair, et al. (2014), Multivalent N-Acetylgalactosamine-Conjugated siRNA Localizes in Hepatocytes and Elicits Robust RNAi-Mediated Gene Silencing. J. Am. Chem. Soc., 136, 16958-16961.
Non-Patent Literature 6: MIHAELA-CARMEN UNCIULEAC and STEWART SHUMAN (2019), RNA 21: 824-832.

## SUMMARY OF THE INVENTION

### PROBLEM TO BE SOLVED BY THE INVENTION

The object of the present invention is to provide a method for efficiently producing an oligonucleotide comprising a complementary portion, such as an siRNA and a heteroduplex oligonucleotide.

### SOLUTION TO PROBLEM

The inventors of the present invention carried out an extensive investigation; as a result, it was found, among other things, that when four or more oligonucleotide raw material fragments corresponding to the fragments of both complementary portions of the oligonucleotide having the target complementary portions was treated with an oligonucleotide ligase, the oligonucleotide having the complementary portion such as a double strand could be directly constructed, and that this could be produced with a higher production efficiency and purity as compared with a linear synthesis method such as a solid phase synthesis. With these findings, the present invention could be accomplished.

Conventionally, comparatively short double strand oligonucleotides such as an siRNA have been produced by a chemical synthesis method because the chemical synthesis thereof is easy and convenient. Specifically, two component oligonucleotide strands thereof are separately synthesized by the chemical synthesis (for example, solid-phase synthesis); and after these are purified, both the strands are annealed to obtain the product. Therefore, there are few reports about the method using the enzymatic condensation. In particular, as for the method for enzymatic synthesis from four or more oligonucleotide raw material fragments, the reported method has been only for production of a double strand oligonucleotide having a comparatively long nucleotide length of 28 or more. Since the nucleotide length of the oligonucleotide raw material fragment to be used for production of a short oligonucleotide is short, it is believed that the annealing ability thereof is low. It is also believed that when the component nucleotide is modified, the annealing ability thereof is further lowered. Under these circumstances, it is believed that the annealing ability of the oligonucleotide raw material fragment is important in the enzymatic oligonucleotide synthetic method using a ligase; thus, there has been no attempt to use the method for producing a shorter oligonucleotide such as a nucleotide length of less than 28 by using four or more oligonucleotide raw material fragments with a ligase.

In spite of these circumstances, the inventors of the present invention carried out an extensive investigation; as a result, it was found that, contrary to the anticipation, when four or more oligonucleotide raw material fragments and a ligase were used, a double strand oligonucleotide having the nucleotide length of less than 28 could be successfully produced without being affected by the annealing ability. It was also found, among other things, that in the case of a short oligonucleotide strand, purity of the produced oligonucleotide was also enhanced. With these findings, the present invention could be achieved.

Namely, the present invention is as follows.

[1] A method for producing a modified oligonucleotide comprising a complementary portion having 11 to 27 nucleotide length, the method comprising forming the modified oligonucleotide by treating four or more oligonucleotide raw material fragments in total in the presence of an oligonucleotide ligase, the four or more oligonucleotide raw material fragments in total correspond to oligonucleotide raw material fragments that are obtained by dividing the modified oligonucleotide at a fragment linking site that satisfies the following conditions (i) to (v):

(i) one or more fragment linking sites are present in the complementary portion in each strand side, and two or more fragment linking sites in total are present in the modified oligonucleotide;
(ii) when the modified oligonucleotide is divided at the fragment linking site, a sticky end is formed in the complementary portion, in which the sticky end has 1 to 10 nucleotide length;
(iii) at least one oligonucleotide raw material fragment has a modified nucleotide;
(iv) four oligonucleotide raw material fragments out of the four or more oligonucleotide raw material fragments in total include the complementary portion having 5 to 25 nucleotide length; and
(v) total nucleotide length of the oligonucleotide raw material fragments corresponding to the complementary portion in each strand side is 11 to 27.

[2] The method according to [1], wherein the sticky end in (ii) has 2 to 6 nucleotide length.
[3] The method according to [1] or [2], wherein a portion other than the sticky end in the complementary portion of the four oligonucleotide raw material fragments defined by (iv) has 4 to 16 nucleotide length.

[4] The method according to any of [1] to [3], wherein the oligonucleotide ligase is an RNA ligase.
[5] The method according to [4], wherein the oligonucleotide ligase is a double strand RNA ligase.
[6] The method according to [5], wherein the double strand RNA ligase is an RNA ligase belonging to the Rnl2 family or the Rnl5 family.
[7] The method according to any of [1] to [6], wherein the modified oligonucleotide comprises a modified nucleotide residue.
[8] The method according to [7], wherein the modified nucleotide residue is a 1', 2', 3', or 4' chemically modified type nucleotide residue, a 5'- or 3'-phosphate-modified type nucleotide residue, a bridging type modified nucleotide residue, a carrier-addition type modified nucleotide residue, or a sugar skeleton-displacement type nucleotide residue.
[9] The method according to [8], wherein the modified nucleotide residue is:

i) the 1', 2', 3', or 4' chemically modified type nucleotide residue having 1', 2', 3', or 4' position thereof displaced with $C_{1-6}$ alkyloxy $C_{1-6}$ alkylene, -O-$C_{1-6}$ alkyl, -O-$C_{6-14}$ aryl, -C-aryl, a halogen atom, -O-$C_{1-6}$ alkyl N-amide $C_{1-6}$ alkylene, -O-$C_{1-6}$ alkyl-($C_{1-6}$ alkyl-)amino-$C_{1-6}$ alkylene, or -O-amino $C_{1-6}$ alkyl (for example, -O-aminopropyl: -O-AP);
ii) the 5'- or 3'-phosphate-modified type nucleotide residue displaced with -O-P(S)$(OH)_2$, -NH-P(O)$(OH)_2$, or -NH-P(S) $(OH)_2$, in which the hydroxide group is optionally displaced with a protection group;
iii) the bridging type modified nucleotide residue having 2' and 4' positions thereof displaced with 2'-O-$C_{1-6}$ alkylene-4', 2'-O-ethylene-4', 2'-O-methyl-substituted methylene-4', 2'-O-$C_{1-6}$ alkylene-O-$C_{1-6}$ alkylene-4', 2'-ON(R)-$C_{1-6}$ alkylene-4' wherein R represents a methyl group, a hydrogen atom, or a benzyl group, 2'-N(R)-C(O)-4', 2'-NH-$C_{1-6}$ alkylene-4', or 2'-$C_{1-6}$ alkylene-4', or a 3' position and a 5' position thereof displaced with 3'-$C_{1-6}$ alkylene-5'; or
iv) a hexitol nucleic acid (HNA) residue, a cyclohexenyl nucleic acid (CeNA) residue, or a morpholino nucleic acid (PMO) residue.

[10] The method according to any of [1] to [9], wherein a total mole ratio of two oligonucleotide raw material fragments arbitrary selected from the oligonucleotide raw material fragments is in a range of 0.5 to 2.
[11] The method according to any of [1] to [10], wherein the oligonucleotide raw material fragments are subjected to a treatment with a monovalent cationic salt whose concentration is 10 mM or less.
[12] The method according to any of [1] to [11], wherein prior to the treatment, a mixed solution of the oligonucleotide raw material fragments is not placed at high temperature and is not cooled down thereafter.
[13] The method according to any of [1] to [12], wherein formation of impurities of the modified oligonucleotide is suppressed.
[14] The method according to any of [1] to [13], wherein the method further comprises purifying the modified oligonucleotide.

## EFFECT OF THE INVENTION

According to the present invention, modified oligonucleotides such as an siRNA and a heteroduplex oligonucleotide can be efficiently produced with high purity.

## BRIEF DESCRIPTION OF DRAWINGS

FIG. 1 is a schematic diagram illustrating one example of the mechanism in the present invention.
FIG. 2-1 (FIG. 2-1 to FIG. 2-6) illustrate plots of siRNA production amount with different concentrations of a T4 RNA ligase 2 when four short natural type RNA fragments are reacted using the T4 RNA ligase 2 in 6 combination patterns (combination numbers of 1 to 6) to produce the same siRNA in Example 1.
FIG. 2-2 is as described above.
FIG. 2-3 is as described above.
FIG. 2-4 is as described above.
FIG. 2-5 is as described above.
FIG. 2-6 is as described above.
FIG. 3-1 (FIG. 3-1 to FIG. 3-4) illustrate plots of the change of siRNA production amount with time at different reaction temperatures when four short natural type RNA fragments in different combinations are reacted by using the T4 RNA ligase 2 in Example 2.

FIG. 3-2 is as described above.

FIG. 3-3 is as described above.

FIG. 3-4 is as described above.

FIG. 4 illustrates HPLC analysis charts to confirm the standard oligonucleotides and the siRNA that is produced from the modified type RNA with different concentrations of the T4 RNA ligase 2 in Example 3.

FIG. 5 illustrates HPLC analysis charts without the enzyme, the chart of the reaction product in the presence of a Deinococcus radiodurans-derived RNA ligase (DraRnl), and the chart of RNA standards (sense strand and antisense strand) in Example 5.

FIG. 6 illustrates a schematic diagram of the reaction product, as well as HPLC analysis charts without the enzyme and the chart of the reaction product in the presence of the T4 RNA ligase 2 in Example 6.

FIG. 7 illustrates a relationship between four oligonucleotide raw material fragments (comprising the oligonucleotide raw material fragment comprising a mismatched base pair) and the double strand modified oligonucleotide that is formed from them.

FIG. 8 illustrates a relationship between five or six oligonucleotide raw material fragments and the double strand modified oligonucleotides that are formed from them.

FIG. 9 illustrates HPLC analysis charts to confirm formation of the double strand modified oligonucleotides in the reaction using 5 or 6 oligonucleotide raw material fragments.

FIG. 10 illustrates a relationship between four oligonucleotide raw material fragments comprising the oligonucleotide raw material fragments having DMTr groups added at the 5' ends thereof and the double strand modified oligonucleotide that is formed from them.

FIG. 11 illustrates a relationship between four oligonucleotide raw material fragments comprising the oligonucleotide raw material fragment having a carrier added at the 5' end thereof and the double strand modified oligonucleotide that is formed from them.

FIG. 12 illustrates a relationship between four oligonucleotide raw material fragments in which phosphate groups are replaced with thiophosphate groups at the linking sites of the nucleotide residues and the double strand modified oligonucleotide that is formed from them.

FIG. 13 illustrates a relationship between four oligonucleotide raw material fragments and the hairpin type modified oligonucleotide that is formed from them.

FIG. 14 illustrates a relationship between four oligonucleotide raw material fragments that are used to compare the effect to the reactivity thereof due to the nucleotide length of the sticky end in the oligonucleotide raw material fragment and the double strand modified oligonucleotide that is formed from them.

FIG. 15 illustrates a relationship between four oligonucleotide raw material fragments that are used to compare the effect of the nucleotide length in the product to the reactivity thereof and the double strand modified oligonucleotide that is formed from them.

FIG. 16 illustrates a relationship between four oligonucleotide raw material fragments that are used to study the initial reaction rate at high substrate concentration and the double strand modified oligonucleotide that is formed from them.

FIG. 17 illustrates a relationship between four oligonucleotide raw material fragments that are used to compare the nucleotide length in the product and the double strand modified oligonucleotide that is formed from them.

## EMBODIMENTS FOR CARRYING OUT THE INVENTION

### Outline of the Present Invention

Hereinafter, the present invention will be explained. In order to help explanation of the present invention, one example of the mechanism in the present invention is schematically illustrated in FIG. 1. It must be noted that this schematic illustration is the example to merely explain the present invention; so, this does not restrict the present invention.

The present invention provides the method for producing a modified oligonucleotide comprising a complementary portion having 11 to 27 nucleotide length (hereinafter, this oligonucleotide is also called "target modified oligonucleotide" and the like). The method of the present invention includes formation of the target modified oligonucleotide by treating, as the raw material, 4 or more oligonucleotide raw material fragments in total in the presence of an oligonucleotide ligase. Hereinafter, the explanation will be made in detail about, among other things, the target modified oligonucleotide that is produced by the method of the present invention, the oligonucleotide raw material fragments and oligonucleotide ligase that are used in the method of the present invention, the individual treatment conditions to carry out the method of the present invention, and the like.

### Target Modified Oligonucleotide

The target modified oligonucleotide that is produced by the method of the present method is the modified oligonucleotide comprising a complementary portion having the nucleotide length of 11 to 27.

"Oligonucleotide" means the oligomer having nucleotide residues as monomer units. Illustrative examples of "oligonucleotide" include an oligo RNA, an oligo DNA, and an RNA-DNA hybrid oligonucleotide.

The oligonucleotide can be classified into "natural type oligonucleotides" and "modified oligonucleotides". "Natural type oligonucleotide" means the oligonucleotide that is composed of nucleotide residues (adenosine (A), guanosine (G), cytidine (C), uridine (U), deoxyadenosine (dA), deoxyguanosine (dG), deoxycytidine (dC), and thymidine (dT); hereinafter, these are called "natural type nucleotide residues") that constitute the polynucleotides (RNA and DNA) included in a cell. "Modified oligonucleotides" mean oligonucleotides other than "natural type nucleotides", and they are the oligonucleotides that include composition elements other than the natural type nucleotide residues (hereinafter, these elements are called "modified residues"). Illustrative examples of the modified residue include a modified nucleotide residue, an amino acid residue, and a linker. Illustrative examples of the modified nucleotide residue include the nucleotide residues having been modified, which will be described later. Amino acids include amino acid derivatives. Illustrative examples of the amino acid include glycine, alanine, valine, leucine, isoleucine, proline, methionine, phenylalanine, tryptophan, serine, threonine, asparagine, glutamine, tyrosine, cysteine, aspartic acid, glutamic acid, histidine, lysine, arginine, and derivatives of them. The amino acid derivative means the amino acid whose arbitrary atom or group therein is displaced with a different atom or group; for example, a hydrogen atom in the amino group, a hydrogen atom, an oxygen atom, or a hydroxy group in the carboxy group, an arbitrary atom or group in a side chain, or a hydrogen atom bonded to the skeleton carbon atom (for example, $\alpha$-, $\beta$-, $\gamma$-, or $\delta$-carbon atom) is displaced with another atom (for example, a halogen atom such as a fluorine atom, a chlorine atom, a bromine atom, or an iodine atom) or with a group (for example, a substituent group after displacement by chemical modification that is going to be described later).

"Modification" in "modified nucleotide residue" includes displacement of the substituent group in the sugar portion (ribose or deoxyribose) in the nucleotide residue, displacement of the sugar portion itself (sugar skeleton) in the nucleotide residue, and modification of the nucleobase portion in the nucleotide residue (for example, displacement of a substituent group in the nucleobase portion).

Illustrative examples of "displacement of the substituent group in the sugar portion in the nucleotide residue" include displacement of 1'-H, 2'-OH (only ribose), 2'-H, 3'-OH, 3'-$NH_2$, 3'-H, 3'-phosphate group, 4'-H, or 5'-phosphate group, or a combination of these. Here, "phosphate group" includes not only -O-P(O)$(OH)_2$ but also the group whose oxygen atom is displaced with a sulfur atom or NH (for example, -O-P(S)$(OH)_2$, -NH-P(O)$(OH)_2$, and -NH-P(S)$(OH)_2$)). "Phosphate group" also includes the phosphate group having the hydroxy group (-OH) thereof displaced with OR* (in the formula, R* represents an organic group such as a protection group of the phosphate group) (for example, a protected phosphate group). Illustrative examples of the displacement like this include the chemical modification at 1', 2', 3', or 4' position (displacement with other substituent group at 1', 2', 3', or 4' position), the modification of the 5'- or the 3'-phosphate group (displacement of the 5'- or the 3'-phosphate group with other substituent group), the bridging type modification (bridging displacement between two of 1', 2', 3', or 4' position), and the carrier-addition type modification (displacement at the position of 1', 2', 3', 4', or 5' with a carrier).

The chemical modification may be done, for example, to enhance a degradation resistance of the oligonucleotide. Illustrative examples of the substituent group after displacement by the chemical modification include $C_{1-6}$ alkyloxy $C_{1-6}$ alkylene (for example, methoxyethyl: MOE), -O-$C_{1-6}$ alkyl (for example, -O-Me), -O-$C_{6-14}$ aryl (for example, -O-phenyl), -C-aryl (for example, - C-phenyl), a halogen atom (for example, a fluorine atom), - O-$C_{1-6}$ alkyl N-amide $C_{1-6}$ alkylene (for example, -O-N-methylacetamide: -O-NMA), -O-$C_{1-6}$ alkyl-($C_{1-6}$ alkyl-)amino-$C_{1-6}$ alkylene (for example, -O-dimethylaminoethoxyethyl: -O-DMAEOE), and -O-amino $C_{1-6}$ alkyl (for example, -O-aminopropyl: -O-AP). The chemical modification is preferably the chemical modification at the 2' position (displacement at the 2' position) and the chemical modification at the 3' position (displacement at the 3' position), while more preferably the chemical modification at the 2' position (displacement at the 2' position). Illustrative examples of the substituent group after displacement by the chemical modification at the 2' position include 2'-$C_{1-6}$ alkyloxy $C_{1-6}$ alkylene (for example, 2'-methoxyethyl), 2'-O-$C_{1-6}$ alkyl (for example, 2'-O-Me), 2'-O-$C_{6-14}$ aryl (for example, 2'-O-phenyl), 2'-C-aryl (for example, 2'-C-phenyl), 2'-halogen atom (for example, 2'-F), 2'-O-$C_{1-6}$ alkyl N-amide $C_{1-6}$ alkylene (for example, 2'-O-N-methylacetamide: 2'-O-NMA), 2'-O-$C_{1-6}$ alkyl-($C_{1-6}$ alkyl-)amino-$C_{1-6}$ alkylene (for example, 2'-O-dimethylaminoethoxyethyl: 2'-O-DMAEOE), and 2'-O-amino $C_{1-6}$ alkyl (for example, 2'-O-aminopropyl: 2'-O-AP). Illustrative examples of the substituent group after displacement by the chemical modification at the 3' position include 3'-O-P(O)$(OH)_2$, 3'-O-P(S)$(OH)_2$, 3'-NH-P(O)$(OH)_2$, 3'-NH-P(S) $(OH)_2$, and the group having the hydroxy group (-OH) in the phosphate group displaced with OR* (in the formula, R* represents an organic group such as a protection group of the phosphate group, which will be described later).

The modification of the 5'- or 3'-phosphate group may be done, for example, to enhance a degradation resistance of the oligonucleotide. Illustrative examples of the modification of the 5'- or 3'-phosphate group include displacement of the phosphate group (-O-P(O)$(OH)_2$) with the group having the oxygen atom in the phosphate group displaced with a sulfur atom or with NH. Illustrative examples of the group like this include -OP(S)$(OH)_2$ (thiophosphate group: phosphorothioate type modification), -NH-P(O)$(OH)_2$, and -NH-P(S)$(OH)_2$. The modification of the 5'- or 3'-phosphate group also includes the group having the hydroxy group (-OH) in the phosphate group displaced with OR* (in the formula, R* represents an organic group such as a protection group of the phosphate group) (for example, a protected phosphate group). Illustrative examples of the protection group of the phosphate group include a trityl (Tr) group, a p-methoxyphenyl diphenylmethyl (MMTr) group, a di(p-methoxyphenyl)phenylmethyl (DMTr) group, and a cyanoethyl ($CN-C_2H_4$-) group.

The bridging type modification may be introduced, for example, to enhance the stability of a steric structure of the nucleotide residue. Illustrative examples of the bridging type modification include the 2',4'-bridging type modification (bridging displacement between 2'-OH and 4'-H) and the 3',5'-bridging type modification (bridging displacement between 3'-H and 5'-H). Illustrative examples of 2',4'-bridging type modification include: displacement of 2'-OH and 4'-H with 2'-O-$C_{1-6}$ alkylene-4' (for example, 2'-O-methylene-4' (locked nucleic acid: LNA), with 2'-O-ethylene-4' (for example, ethylene-bridged nucleic acid: ENA), and with 2'-O-methyl-substituted methylene-4' (for example, constrained ethyl-bridged nucleic acid: one kind of BNA (cEt-BNA); displacement of 2'-OH with 4'-H with 2'-O-$C_{1-6}$ alkylene-O-$C_{1-6}$ alkylene-4' (for example, 2'-O-methylene-O-methylene-4' (bridged nucleic acid: one kind of BNA ($BNA^{coc}$)); displacement of 2'-OH and 4'-H with 2'-ON(R)-$C_{1-6}$ alkylene-4' (for example, 2'-O-N(R)-methylene-4' (bridged nucleic acid: one kind of BNA ($BNA^{NC}$); here, R represents a methyl group, a hydrogen atom, or a benzyl group); displacement of 2'-$NH_2$ and 4'-H with 2'-N(R)-C(O)-4' (for example, 2-N(methyl)-C(O)-4' (amide-bridged nucleic acid: AmNA)); displacement of 2'-$NH_2$ and 4'-H with 2'-NH-$C_{1-6}$ alkylene-4' (for example, 2'-NH-methylene-4'); and displacement of 2'-H and 4'-H with 2'-$C_{1-6}$ alkylene-4' (for example, 2'-methyl-substitued ethylene-4'). Illustrative examples of 3',5'-bridging type modification include displacement of 3'-H and 5'-H with 3'-$C_{1-6}$ alkylene-5' (for example, 3'-ethylene-5' (bicyclo-nucleic acid: Bc nucleic acid), one kind of Bc nucleic acids: tc nucleic acid, and the like).

The carrier in the carrier-addition type modification may be those that can improve the stability, target directivity, medical efficacy, and the like of the target modified oligonucleotide, or add these properties to the target modified oligonucleotide. These carriers may be chosen as appropriate from heretofore known carriers in accordance with the use purpose thereof. Illustrative examples of the carrier include N-acetylgalactosamine (GalNAc), peptide, phosphoric acid, cholesterol, tocopherol, a fat chain, and folic acid. The site of the addition in the carrier-addition type modification is preferably 3' or 5', which corresponds to the end of the target modified oligonucleotide.

Illustrative examples of the modified nucleotide residue including "displacement of the sugar portion itself in the nucleotide residue" (sugar skeleton-displacement type nucleotide residue) include the nucleotide residue including displacement of the 5-membered ring sugar in the oligonucleotide with the 6-membered ring quasi-sugar such as hexitol nucleic acid (HNA) and cyclohexenyl nucleic acid (CeNA). Illustrative examples of the modified nucleotide residue including "displacement of the sugar portion itself in the nucleotide residue" further include a morpholino nucleic acid (PMO) residue, which is a nucleotide-like artificial compound having a morpholino ring structure that is not degradated by an enzyme in a living body (for example, a nuclease such as RNase) and does not induce an immune response.

Illustrative examples of "modification of the nucleobase portion in the nucleotide residue" include the nucleotide residue having the nucleobase portion thereof displaced with an alkyl group (for example, the cytosine group at the 5-position thereof displaced with a methyl group).

"Oligonucleotide comprising a complementary portion" means the oligonucleotide comprising a paired structure of the complementary nucleotide sequences with each other. Illustrative examples of "oligonucleotide comprising a complementary portion" include a double strand oligonucleotide and a single strand oligonucleotide having a double strand-like structure (for example, loop type oligonucleotides such as a hairpin type oligonucleotide and a dumbbell type oligonucleotide). The double strand oligonucleotide may be the double strand oligonucleotide in which each strand is the above-mentioned oligonucleotide. Illustrative examples thereof include a double strand oligo RNA, a double strand oligo DNA, a heteroduplex oligonucleotide composed of an oligo RNA and an oligo DNA, a double strand oligonucleotide composed of an oligo RNA and an RNA-DNA hybrid oligonucleotide, a double strand oligonucleotide composed of an oligo DNA and an RNA-DNA hybrid oligonucleotide, and a double strand oligonucleotide composed of RNA-DNA hybrid oligonucleotides. Illustrative examples of the double strand oligonucleotide include an siRNA and a heteroduplex oligonucleotide. In the oligonucleotide comprising the complementary portion, the portion where the complementary nucleotide sequences are paired is called "complementary portion". The term "complementary portion" means not only the complementary portion in the oligonucleotide comprising the complementary portion, but also the portion, in the oligonucleotide raw material fragment, corresponding to the complementary portion in the oligonucleotide comprising the complementary portion when the oligonucleotide comprising the complementary portion is divided to the oligonucleotide raw material fragments. For the sake of convenience, an arbitrary complementary nucleotide sequence in the complementary portion is sometimes called "sense strand", and the other complementary nucleotide sequence is sometimes called "antisense strand". In the present invention, the terms "sense" and "antisense" are merely the names for convenience to distinguish an arbitrary one from the other one in the complementary portion, not intending the biological significance (especially, significance in RNAi). The oligonucleotide comprising the complementary portion may include or does not necessarily include a loop portion. "Loop portion" means a linker to link between the sense side and the antisense side in the complementary portion at the same end sides (for example, the 5' end and the 3' end). The oligonucleotide comprising the complementary portion is used especially for the action of the post-transcriptional gene silencing (for example, RNA intervention (RNAi)).

The target modified oligonucleotide includes the above-mentioned modified residue in the complementary portion thereof. Illustrative examples of the target modified oligonucleotide include the double strand oligonucleotide and loop type oligonucleotide that include the modified nucleotide residue (for example, the double strand oligonucleotide and loop-type oligonucleotide that include the modified nucleotide residue in the complementary portion) and the loop type oligonucleotide comprising the modified nucleotide residue or the residue other than the nucleotide residue (for example, an amino acid residue, a linker, and the like) in the loop portion (for example, International Publication No. 2012-005368). In the target modified oligonucleotide, part of the nucleotide residues may be the modified nucleotide, or entire of the nucleotide residues may be the modified nucleotide; but in the case where "modified nucleotide residue" is a morpholino nucleic acid (PMO) residue, preferably part of the nucleotide residues is the morpholino nucleic acid (PMO) residue in the target modified oligonucleotide. In addition, the target modified oligonucleotide includes: a gapmer, which is the oligonucleotide that has modified nucleotide residues in both the ends of the sequence thereof and has in the center portion of the sequence thereof a gap region that receives a recognition by an RNase; a mixmer, the oligonucleotide in which modified nucleotide residues are included as a mixture in the sequence thereof; and the oligonucleotide that does not induce the RNase activity such as a totally modified oligonucleotide in which all the nucleotide residues in the sequence thereof are the modified nucleotide residues.

In the present invention, the complementary portion in the target modified oligonucleotide has the nucleotide length of 11 to 27 (for example, the nucleotide length of 12 to 27, the nucleotide length of 15 to 27, or the nucleotide length of 18 to 27). For example, in the case where the target modified oligonucleotide is the double strand modified oligonucleotide formed of only the complementary portion, the nucleotide length thereof may be 11 to 27. Alternatively, the target modified oligonucleotide may have, in addition to the complementary portion, a non-complementary portion. In this case, the non-complementary portion may have the nucleotide length of 1 to 16, for example, the nucleotide length of 1 to 10, preferably the nucleotide length of 1 to 5, while still more preferably the nucleotide length of 1, 2, or 3. In the target modified oligonucleotide having the non-complementary portion in addition to the complementary portion having the nucleotide length of 11 to 27, the complementary portion having the nucleotide length of 11 to 27 may be not only in a continuous form, but also in a noncontinuous form in which the complementary portion is separated by a mismatched base pair as the non-complementary portion.

The total number of the residues in the target modified oligonucleotide may be chosen as appropriate in view of the function of the target modified oligonucleotide and the individual conditions in the method of the present invention. The total number of the residues in the target modified oligonucleotide may also be, for example, 24 to 74.

Oligonucleotide Raw Material Fragment

The four or more oligonucleotide raw material fragments in total that are used as the raw materials in the method of the present invention may be designed to correspond to oligonucleotide raw material fragments that are obtained by dividing the target modified oligonucleotide at a fragment linking site (this is also called "cleaving site") that satisfies the following (i) to (v) conditions:

(i) one or more fragment linking sites are present in the complementary portion in each strand side, and two or more fragment linking sites in total are present in the modified oligonucleotide;
(ii) when the modified oligonucleotide is divided at the fragment linking site, a sticky end (this is also called "cohesive end") is formed in the complementary portion, in which the sticky end has 1 to 10 nucleotide length;
(iii) at least one oligonucleotide raw material fragment has a modified nucleotide;
(iv) four oligonucleotide raw material fragments out of the four or more oligonucleotide raw material fragments in total include the complementary portion having 5 to 25 nucleotide length; and
(v) the total nucleotide length of the oligonucleotide raw material fragments corresponding to the complementary portion in each strand side is 11 to 27.

The number of the oligonucleotide raw material fragment is four or more, while preferably in the range of 4 to 6 (4, 5, or 6). The number of the oligonucleotide raw material fragment may be characterized from a viewpoint of the number mainly corresponding to the sense strand and the antisense strand that constitute the target modified oligonucleotide (mainly double strand nucleic acid). From the condition (i), it can be understood that the number of the oligonucleotide raw material fragments mainly corresponding to the sense strand and the antisense strand is 2 or more in each strand. The number of the oligonucleotide raw material fragments corresponding to the sense strand and the antisense strand may be three fragments or four fragments in each, while preferably two fragments or three fragments. The sticky end in the condition (ii) may be any of the 5' sticky end and the 3' sticky end. "Complementary portion" in the condition (iv) means the portion in the oligonucleotide raw material fragment that corresponds to the complementary portion in the target modified oligonucleotide. In the present invention, the terms "fragment linking site" and "cleaving site" have the same meaning. "Fragment linking site" ("cleaving site") means the site that is used for the sake of convenience to design the combination of the oligonucleotide raw material fragments; so, this does not mean the site that is actually cleaved in the method of the present invention. The four oligonucleotide raw material fragments in (iv) may also be designed such that the target modified oligonucleotide may include the complementary portion having the nucleotide length of preferably 5 to 25, and more preferably 5 to 20, while still more preferably 5 to 17.

The nucleotide length in the "complementary portion" in the condition (iv) only needs to be long enough to form the pair; so, the nucleotide length thereof may be one or more. In the synthesis of the oligonucleotide, the purity, yield, and production efficiency of the product can decrease as the nucleotide length increases; thus, the four oligonucleotide raw material fragments out of the entire oligonucleotide raw material fragments are designed preferably such that the nucleotide length of the complementary portion thereof may be 17 or less. The nucleotide length of the two strands that constitute the complementary portion is preferably 5 to 25 (for example, 5 to 22 nucleotide length, 5 to 20 nucleotide length, 5 to 17 nucleotide length, 8 to 25 nucleotide length, 8 to 22 nucleotide length, 8 to 20 nucleotide length, or 8 to 17 nucleotide length).

The nucleotide length of the sticky end is, for example, 1 to 10, preferably 1 to 8, and more preferably 1 to 6, while still more preferably, 2 to 6, 3 to 6, or 4 to 6.

The number of the nucleotide length in "complementary portion" in the condition (iv) and the number of the nucleotide length in the sticky end are determined such that the above-mentioned numerical ranges may be satisfied and that these numbers conform with each other. For example, when the nucleotide length in the sticky end is 5, the nucleotide length in the complementary portion to form the sticky end may be 6 to 25; for example, when the nucleotide length of the sticky end is 6, the nucleotide length of the complementary portion to form the sticky end may be 7 to 25.

The nucleotide length of the portion other than the sticky end in "complementary portion" in the four oligonucleotide raw material fragments that are stipulated in the condition (iv) is preferably, 4 to 24, 4 to 21, 4 to 19, or 4 to 16.

Under a certain embodiment, the nucleotide length of each of the four oligonucleotide raw material fragments that are stipulated in the condition (iv) may be 5 or more, preferably 6 or more, more preferably 7 or more, and still more preferably 8 or more, while especially preferably 9 or more. The nucleotide length of each of the four oligonucleotide raw material fragments as mentioned above may also be 19 or less, preferably 18 or less, more preferably 17 or less, and still more preferably 16 or less, while especially preferably 15 or less. The nucleotide length of each of the four oligonucleotide raw material fragments as mentioned above may be in the range of 5 to 19, preferably in the range of 6 to 18, more preferably in the range of 7 to 17, and still more preferably in the range of 8 to 16, while especially preferably in the range of 9 to 15.

The 5' end of the oligonucleotide raw material fragment corresponding to the 5' end of the target modified oligonucleotide may be as it is in the form of the 5'-phosphate group, or may be displaced with 5'-OH, or may be modified with a 5' phosphate modifying group, or may have the same structure as the 5' end of target modified oligonucleotide. The modification of the 5'-phosphate group may be, for example, those mentioned above. The 5' end of the oligonucleotide raw material fragments other than these is, from a viewpoint of the linking reaction by an oligonucleotide ligase, preferably as it is in the form of the 5'-phosphate group. The 3' end of the oligonucleotide raw material fragment corresponding to the 3' end of the target modified oligonucleotide may be as it is in the form of the 3'-OH, or may be modified with a 3' phosphate modifying group, or may have the same structure as the 3' end of target modified oligonucleotide. The modification of the 3'-phosphate group may be, for example, those mentioned above. The 3' end of the oligonucleotide raw material fragments other than these is, from a viewpoint of the linking reaction by an oligonucleotide ligase, preferably as it is in the form of the 3'-OH.

The oligonucleotide raw material fragment may be in the free form, or in the composited form, or in the fixed form.

The oligonucleotide raw material fragment may be produced by heretofore known chemical synthesis methods or enzymatic synthesis methods. Illustrative examples of the chemical synthesis method include a solid phase synthesis method and a liquid phase synthesis method, such as those methods described in International Publication No. 2012-157723 and International Publication No. 2005-070859.

When addition of a functional group to the target modified oligonucleotide is desired, the oligonucleotide raw material fragment may be added with the functional group in the corresponding part thereof.

Ligase Treatment

The oligonucleotide ligase is the enzyme to link the oligonucleotide raw material fragments with each other. In the method of the present invention, by the catalytic action of the oligonucleotide ligase, the oligonucleotide raw material fragments are linked with each other at "cleaving site" (This is also called "linking site") to form the target modified oligonucleotide. Illustrative examples of the oligonucleotide ligase include an RNA ligase and a DNA ligase. The RNA ligase may be any of a single strand RNA ligase and a double strand RNA ligase, and the double strand RNA ligase is preferable. Illustrative examples of the double strand RNA ligase include the RNA ligases belonging to the Rnl2 family (this is also called "RNA ligase 2") and the RNA ligases belonging to the Rnl5 family. RNA ligases derived from any biological and virus species may be used so far as the purpose of the present invention can be achieved; for example, an RNA ligase derived from a T4 phage (T4 RNA ligase 1 and T4 RNA ligase 2) may be used. DNA ligases derived from any biological and virus species may be used so far as the purpose of the present invention can be achieved; for example, a DNA ligase derived from a T4 phage may be used.

Treatment in the presence of the oligonucleotide ligase (hereinafter, this is called "ligase treatment") is the reaction to link the oligonucleotide raw material fragments by the catalytic action of the oligonucleotide ligase. Operation of the ligase treatment is mixing of the oligonucleotide raw material fragments with the oligonucleotide ligase. In the ligase treatment, the linking reaction may be carried out in a single stage by mixing all the oligonucleotide raw material fragments with the oligonucleotide ligase. The ligase treatment may also be carried out as a multi-stage linking reaction, in which after the linking reaction is carried out by mixing part of the oligonucleotide raw material fragments with the oligonucleotide ligase, the next linking reaction is carried out by mixing the rest of the oligonucleotide raw material fragments with the reactant. Mixing may be conducted by adding the oligonucleotide ligase into the oligonucleotide raw material fragments, or by adding the oligonucleotide raw material fragments into the system that contains the oligonucleotide ligase, or by adding the oligonucleotide raw material fragments and the oligonucleotide ligase into the system to carry out the reaction.

An aqueous solution may be used as the system in which the ligase treatment is carried out. The aqueous solution is preferably a buffer solution. Illustrative examples of the buffer solution include a phosphate buffer solution, a Tris buffer solution, a carbonate buffer solution, an acetate buffer solution, and a citrate buffer solution. Here, pH may be, for example, in the range of about 5 to about 9. For example, when concentration of the oligonucleotide raw material fragments in the ligase treatment is high, pH may be in the range of 7.5 to 9.0 (for example, 8.0 to 8.5).

The concentration of each of the oligonucleotide raw material fragments in the ligase treatment is preferably within the range in which the oligonucleotide raw material fragments can be dissolved and the concentration thereof is high enough to produce the target modified oligonucleotide. The concentration of each of the oligonucleotide raw material fragments may be, for example, 1 μM or more, 10 μM or more, 50 μM or more, 100 μM or more, 300 μM or more, 500 μM or more, or 1,000 μM or more. Also, the concentration of each of the oligonucleotide raw material fragments may be, for example, 1 M, 100 mM, or 10 mM or less. Especially when a large quantity of the target modified oligonucleotide is desired to be efficiently produced, each of the oligonucleotide raw material fragments is preferably used with the concentration of 100 μM or more within the above-mentioned concentration range and with the pH of 7.5 to 9.0 within the above-mentioned pH range.

From a viewpoint to enhance the production efficiency by lowering the unreacted oligonucleotide raw material fragments, the mole numbers of all the oligonucleotide raw material fragments in the ligase treatment are preferably almost the same. In order to have almost the same mole number in all the oligonucleotide raw material fragments, a total mole ratio of two oligonucleotide raw material fragments arbitrarily selected from the 4 or more oligonucleotide raw material fragments in total are, for example, in the range of 0.5 to 2, preferably in the range of 1/1.8 to 1.8, more preferably in the range of 1/1.5 to 1.5, and still more preferably in the range of 1/1.2 to 1.2, while especially preferably in the range of 1/1.1 to 1.1.

Concentration of the oligonucleotide ligase in the ligase treatment may be high enough to produce the target modified oligonucleotide. The concentration of the oligonucleotide ligase may be, for example, 0.01 U/μL or more, preferably 0.02 U/μL or more, and more preferably 0.03 U/μL or more, while still more preferably 0.04 U/μL or more. The concentration of the oligonucleotide ligase may be, for example, 1 U/μL or less, preferably 0.5 U/μL or less, and more preferably 0.2 U/μL or less, while still more preferably 0.1 U/μL or less. More specifically, the concentration of the oligonucleotide ligase may be, for example, in the range of 0.01 to 1 U/μL, preferably in the range of 0.02 to 0.5 U/μL, and more preferably in the range of 0.03 to 0.2 U/μL, while still more preferably in the range of 0.04 to 0.1 U/μL.

The system in which the ligase treatment is carried out may include a cofactor of the oligonucleotide ligase. Illustrative examples of the cofactor of the oligonucleotide ligase include ATP and divalent metal salts (for example, magnesium salts such as magnesium chloride). The system in which the processing is carried out may include a stabilizer of the oligonucleotide ligase. Illustrative examples of the stabilizer of the oligonucleotide ligase include antioxidants (for example, reducing agents such as dithiothreitol and mercapto ethanol). In order to keep the stability of the enzyme and to facilitate the reaction rate, the system in which the ligase treatment is carried out may include a surfactant. Illustrative examples of the surfactant include a nonionic surfactant (for example, Triton series surfactants such as Triton X-100) and an ionic surfactant. Illustrative examples of the ionic surfactant include a cationic surfactant, an anionic surfactant, and an amphoteric surfactant. Furthermore, in order to facilitate the reaction rate, the system in which the ligase treatment is carried out may include polyethyleneglycol.

The system in which the ligase treatment is carried out may include a monovalent cationic salt with a low concentration or does not necessarily substantially include a monovalent cationic salt. The concentration of the monovalent cationic salt in the system in which the treatment is carried out may be, for example, 10 mM or less, preferably 1 mM or less, and more preferably 0.1 mM or less, while still more preferably 0.01 mM or less. Especially preferably, the system in which the treatment is carried out does not necessarily substantially include the monovalent cationic salt. Illustrative examples of the monovalent cationic salt include a salt of a monovalent cation such as a lithium ion, a sodium ion, a potassium ion, a rubidium ion, a cesium ion, or an ammonium ion with an anion such as a fluoride ion, a chloride ion, a bromide ion, or an iodide ion.

The temperature in the ligase treatment may be any so far as the oligonucleotide ligase can be sufficiently activated under the temperature. The temperature like this may be, for example, in the range of 2 to 50°C, and preferably in the range of 16 to 50°C, while more preferably in the range of 25 to 50°C.

The time for carrying out the ligase treatment is not restricted so far as it is long enough to produce the target modified oligonucleotide. The time for this may be, for example, in the range of 1 to 72 hours.

According to the present invention, in the target modified oligonucleotide, formation of or contamination by an N-1mer, an N+1mer, and the like, which are the impure substances having the nucleotide length other than the target nucleotide length, can be suppressed. For example, in the present invention, the target modified oligonucleotide is produced as a single double strand nucleic acid (for example, an siRNA and a heteroduplex oligonucleotide). The sense strand and antisense strand that constitute the single double strand nucleic acid have the nucleotide lengths of N and M, respectively. The N and M nucleotide lengths each are independently in the range of 11 to 30 (for example, 18 to 30 nucleotide length). The N and M nucleotide lengths each may also be independently in the range of 11 to 27 (for example, 18 to 27 nucleotide length). In the present invention, the impure substances of the target modified oligonucleotide mean nuclear acid contaminants other than the target modified oligonucleotide. The sense strand and antisense strand that constitute the nucleic acid contaminants do not have the nucleotide lengths of N and M, respectively, but have the nucleotide lengths of $(N\pm\alpha)$ and M, the nucleotide lengths of N and $(M\pm\beta)$, or the nucleotide lengths of $(N\pm\alpha)$ and $(M\pm\beta)$, respectively. Here, N and M mean the same as those explained above, and $\alpha$ and $\beta$ are, for example, 1, 2, or 3.

Other Arbitrary Process

The method of the present invention may include a synthesis process to synthesize the oligonucleotide raw material fragments (for example, chemical syntheses such as the solid phase synthesis). In the method of the present invention, formation of the nucleic acid contaminants other than the target modified oligonucleotide can be suppressed; thus, purification of the target modified oligonucleotide from the sample of the synthesized oligonucleotide raw material fragments may be omitted. In the method of the present invention, however, even when purification of the target modified oligonucleotide is carried out, because formation of the double strand nucleic acid contaminants (impure substances) due to small amounts of substances other than the target oligonucleotide raw material fragments that can remain after purification can be suppressed, the purification of the target modified oligonucleotide may also be carried out. The purification like this may be carried out, for example, by a chromatography method (for example, HPLC and IEX) and a gel filtration method.

The method of the present invention may include a reaction termination process after the ligase treatment process. Illustrative examples of the reaction termination process include the inactivation treatment of the oligonucleotide ligase by a high-temperature treatment (for example, 80°C) or by addition of an acid, an alkali, or an organic solvent, and a removal of a metal ion as the cofactor by addition of a chelating agent such as EDTA. In addition, illustrative examples of the method include a method in which the reaction is carried out using an immobilized enzyme followed by removal of the enzyme from the reaction solution by a membrane separation.

The method of the present invention may include a process to purify the target modified oligonucleotide after the ligase treatment process. This process may be carried out by an arbitrary, suitable method such as a chromatography method (for example, HPLC) or a gel permeation method.

In the case that the oligonucleotide raw material fragments are annealed, in general, in order to bring the oligonucleotide raw material fragments to a denatured state (non-paired state), the mixed solution of the oligonucleotide raw material fragments is heated to a high temperature; then, in order to pair the complementary nucleotide sequences with each other, in many instances the mixed solution of the oligonucleotide raw material fragments having been heated to the high temperature is gradually cooled by an air or the like. In the method of the present invention, however, without carrying out the heating operation to the high temperature for denaturing and the cooling treatment for pairing (heating-cooling process), the target modified oligonucleotide can be produced with a simplified operation. Heating to the high temperature may be done, for example, by keeping the mixed solution of the oligonucleotide raw material fragments at the temperature of, for example, 65°C or more, 70°C or more, 75°C or more, 80°C or more, 85°C or more, 90°C or more, 95°C or more, or 100°C or more (for the period of 5 minutes or more, or 10 minutes or more). Cooling may be done, for example, by statically leaving the mixed solution of the oligonucleotide raw material fragments at room temperature (for example, in the temperature range of 15 to 25°C, or 20 to 25°C) or at a prescribed temperature (for example, at 37°C) (for example, 5 hours or more), or by keeping the mixed solution of the oligonucleotide raw material fragments at a prescribed temperature (for example, at 37°C) (for example, 15 minutes or more).

In order to omit the heating-cooling process, the time during which the mixed solution of the oligonucleotide raw material fragments is kept at the high temperature before the ligase treatment process may be controlled to be, for example, less than 5 minutes, 4.5 minutes or less, 4 minutes or less, 3.5 minutes or less, 3 minutes or less, 2.5 minutes or less, 2 minutes or less, 1.5 minutes or less, 1 minute or less, or 0.5 minute or less.

In the method of the present invention, in order to omit the heating-cooling process, the solution including the oligonucleotide raw material fragments may be kept in the temperature range of 2 to 50°C from the time to mix the oligonucleotide raw material fragments in a solution till to the time of carrying out the ligase treating process. Namely, in the method of the present invention, any mixing of all the oligonucleotide raw material fragments and the oligonucleotide ligase with any combination thereof as described before, the interval between any mixing, as well as the ligase reaction may be carried out under the condition of 2 to 50°C. In the embodiments like this, the method of the present invention includes the following:

(1) all the mixing of all the oligonucleotide raw material fragments included in a different system in any afore-mentioned combination and mixing thereof with the oligonucleotide ligase are carried out in the temperature range of 2 to 50°C; and in the interval between all the mixing, the mixing is carried out under the condition where the mixture is kept in the temperature range of 2 to 50°C to obtain the mixed solution; and
(2) the mixed solution is caused to react with keeping the temperature in the range of 2 to 50°C to obtain the solution including the target modified oligonucleotide.

In this embodiment, all the oligonucleotide raw material fragments included in the afore-mentioned combination are obtained in a different system. In this embodiment, the present invention may be carried out, for example, in such a way that the oligonucleotide raw material fragments are mixed in the temperature range of 2 to 50°C to obtain an oligonucleotide raw material fragments mixture, followed by mixing this oligonucleotide raw material fragments mixture with the oligonucleotide ligase in the temperature range of 2 to 50°C. In this embodiment, the present invention may also be carried out, for example, in such a way that the oligonucleotide raw material fragments are successively added into the solution including the oligonucleotide ligase in the temperature range of 2 to 50°C.

The method of the present invention may be used, for example, for industrial production of the target, modified oligonucleotide in a large scale.

EXAMPLES

Next, the present invention will be explained in more detail by Examples, but the present invention is not limited to these Examples.

Example 1: Comparison of Combination Patterns of Fragments Using Natural Type RNA

1) Synthesis of Substrates and Product Standards

In the enzymatic synthesis of an siRNA from four short natural type RNA fragments, the effect of the nucleotide length of the fragments was evaluated. The double strand composed of RNA1-S (21mer) and RNA1-A (23mer) as listed in Table 1 (hereinafter, these are called the sense strand and the antisense strand, respectively) was chosen as the target siRNA. Eighteen RNA fragments listed in Table 1 were synthesized, and 6 combination patterns of the fragments listed in Table 2 were evaluated.

Table 1. Natural type RNA used for evaluation

| Usage | Name | Sequence (5'→3') | Nucleotide length | SEQ ID NO |
|---|---|---|---|---|
| Standard (sense strand) | RNAl-S | AACAGUGUUCUUGCUCUAUAA | 21 | 1 |
| Standard (antisense strand) | RNA1-A | UUAUAGAGCAAGAACACUGUUUU | 23 | 2 |

(continued)

| Usage | Name | Sequence (5'→3') | Nucleotide length | SEQ ID NO |
|---|---|---|---|---|
| Substrate fragment for sense strand | RNAl-S-9U | AACAGUGUU | 9 | - |
| | RNAl-S-9D | Pho-CUUGCUCUAUAA | 12 | 3 |
| | RNAT-S-10U | AACAGUGUUC | 10 | 4 |
| | RNAT-S-10D | Pho-UUGCUCUAUAA | 11 | 5 |
| | RNA1-S-11U | AACAGUGUUCU | 11 | 6 |
| | RNA1-S-11D | Pho-UGCUCUAUAA | 10 | 7 |
| | RNA1-S-12U | AACAGUGUUCUU | 12 | 8 |
| | RNA1-S-12D | Pho-GCUCUAUAA | 9 | - |
| Substrate fragment for antisense strand | RNA1-A-10U | UUAUAGAGCA | 10 | 9 |
| | RNA1-A-10D | Pho-AGAACACUGUUUU | 13 | 10 |
| | RNA1-A-11U | UUAUAGAGCAA | 11 | 11 |
| | RNA1-A-11D | Pho-GAACACUGUUUU | 12 | 12 |
| | RNA1-A-12U | UUAUAGAGCAAG | 12 | 13 |
| | RNA1-A-12D | Pho-AACACUGUUUU | 11 | 14 |
| | RNA1-A-13U | UUAUAGAGCAAGA | 13 | 15 |
| | RNA1-A-13D | Pho-ACACUGUUUU | 10 | 16 |
| English capital letter: RNA;<br>Pho: modification at 5'-end by phosphate group. | | | | |

Table 2. Combination of natural type RNA fragments

| No. | Sense strand | | Antisense strand | | Sticky end | |
|---|---|---|---|---|---|---|
| | 5' side fragment | 3' side fragment | 5' side fragment | 3' side fragment | Nucleotide length | Sequence[1)] |
| 1 | RNA1-S-12U | RNA1-S-12D | NA1-A-13U | RNA1-A-13D | 4 | UCUU |
| 2 | RNA1-S-12U | RNA1-S-12D | RNA1-A-12U | RNA1-A-12D | 3 | CUU |
| 3 | RNA1-S-12U | RNA1-S-12D | RNA1-A-11U | RNA1-A-11D | 2 | UU |
| 4 | RNA1-S-11U | RNA1-S-11D | NA1-A-13U | RNA1-A-13D | 3 | UCU |
| 5 | RNA1-S-10U | RNA1-S-10D | NA1-A-13U | RNA1-A-13D | 2 | UC |
| 6 | RNA1-S-9U | RNA1-S-9D | RNA1-A-10U | RNA1-A-10D | 2 | CU |
| 1) This sequence indicates the sequence of sticky end site for sense strand. | | | | | | |

2) Ligation Reaction by T4 RNA Ligase 2

The reaction was carried out using 4 oligonucleotide fragments by T4 RNA ligase 2 (New England Biolabs). The reaction solution having the composition of 50 mM of Tris-HCl, 2 mM of $MgCl_2$, 1 mM of dithiothreitol, 400 $\mu$M of ATP, pH of 7.5, and 10 μM of each RNA fragment, with 10 μL as the volume of the reaction solution was used. The concentrations of the added enzyme were 0.025, 0.05, 0.1, and 0.2 U/μL, respectively, to compare the product concentration. By using a thermal cycler, the reaction was conducted at 25°C for 1 hour, and then, the reaction was terminated by heating the reaction solution at 80°C for 5 minutes.

3) Analysis by HPLC

The reaction solution was analyzed by HPLC using Xbridge Oligonucleotide BEH C18 Column (Waters, 2.5 μm, 4.6 mm x 50 mm). The analysis was conducted with the condition: the column temperature of 60°C, the detection wavelength of 254 nm, the injection amount of 10 μL, and the flow rate of 0.4 mL/min. The linear gradient using the mobile phase of the eluting solution A (hexafluoroisopropanol-triethylamine) and the eluting solution B (methanol) was used for the analysis. The standards of the sense strand and the antisense strand were similarly analyzed to quantify the concentrations of the ligation products.

4) Results

Accumulations of the sense strand and the antisense strand in the combinations of the nucleotide length of each fragment are illustrated in FIG. 2. The ligation products were hardly accumulated in the combination 3; but in the rest of the combinations, it was observed that accumulations of the ligation products were increased with increase of the enzyme concentration.

Example 2: Evaluation of Effects of Reaction Temperature in Ligation Reaction of Natural Type RNA

Effects of the reaction temperature in the short strand ligation reaction were evaluated. The oligonucleotides No. 1 in Table 2 were used as the substrates, and the T4 RNA ligase 2 was used to cause reaction. The reaction solution having the composition of 50 mM of Tris-HCl, 2 mM of $MgCl_2$, 1 mM of dithiothreitol, 400 μM of ATP, pH of 7.5, 0.2 U/μL of the enzyme concentration, and 10 μM of each RNA fragment, with 10 μL as the volume of the reaction solution was used. The reaction temperatures were set at 16°C, 25°C, 30°C, and 37°C; and after 1, 2, and 4 hours of each reaction, the reaction solution was heated at 80°C for 5 minutes to terminate the reaction. The concentrations of the ligation products included in the reaction solution were analyzed by HPLC with the condition described in Example 1.

The results are illustrated in FIG. 3. At the reaction temperature of 25°C or more, about 10 μM of the ligation products were accumulated after one hour of the reaction in both the sense strand and the antisense strand. On the other hand, the formation rate of the ligation product especially in the sense strand was slow at 16°C as compared with the temperatures of 25°C or more.

Example 3: Confirmation of Progress of the Reaction Using Modified RNA

1) Syntheses of Substrates and Product Standards

In the modified oligonucleotide, progress of the enzymatic ligation reaction of the siRNA from four fragments was evaluated. The double strand composed of the sense strand (MOD1-S) and the antisense strand (MOD1-A) as listed in Table 3 was chosen as the target siRNA. This siRNA has the same nucleotide sequence as the natural RNA that was used in Example 1 and Example 2, but all the residues are modified with 2'-F or 2'-O-methyl, and part of the phosphate group is displaced with a thiophosphate group. Also, four fragments illustrated in Table 3 were synthesized as the respective fragments. The nucleotide sequences of these four fragments are the same as the combination No. 1 in Table 2.

Table 3. Modified type RNA used for evaluation

| Usage | Name | Sequence | SEQ ID NO |
|---|---|---|---|
| Sense strand standard | MOD1-S | A(F)^A(Me)^C(F)A(Me)G(F)U(Me)G(F)U(Me)U(F)C(F)U(F)U(Me)G(F)C(Me)U(F)C(Me)U(F)A(Me)U(F)A(Me)A(F) | 1 |
| Antisense strand standard | MOD1-A | U(Me)^U(F)^A(Me)U(F)A(Me)G(F)A(Me)G(F)C(Me)A(F)A(Me)G(Me)A(Me)A(F)C(Me)A(F)C(Me)U(F)G(Me)U(F)U(Me)^U(Me)^U(Me) | 2 |

(continued)

| Usage | Name | Sequence | SEQ ID NO |
|---|---|---|---|
| Sense strand 5' side fragment | MOD1-S-12U | A(F)^A(Me)^C(F)A(Me)G(F)U(Me)G(F)U(Me)U(F)C(F)U(F)U(Me) | 8 |
| Sense strand 3' side fragment | MOD1-S-12D | Pho-G(F)C(Me)U(F)C(Me)U(F)A(Me)U(F)A(Me)A(F) | - |
| Antisense strand 5' side fragment | MOD1-A-13U | U(Me)^U(F)^A(Me)U(F)A(Me)G(F)A(Me)G(F)C(Me)A(F)A(Me)G(Me)A(Me) | 15 |
| Antisense strand 3' side fragment | MOD1-A-13D | Pho-A(F)C(Me)A(F)C(Me)U(F)G(Me)U(F)U(Me)^U(Me)^U(Me) | 16 |
| English capital letter: RNA;<br>Pho: modification at 5'-end by phosphate group;<br>(F) : modification by 2'-fluoro group;<br>(Me) : modification by 2'-O-methyl group;<br>^: Substitution of phosphate group with thiophosphate group. | | | |

2) Ligation Reaction by T4 RNA Ligase 2

The reaction was carried out using four modified RNA fragments by the T4 RNA ligase 2. The reaction solution having the composition of 50 mM of Tris-HCl, 2 mM of $MgCl_2$, 1 mM of dithiothreitol, 400 μM of ATP, pH of 7.5, and 10 μM of each modified RNA fragment, with 50 μL as the volume of the reaction solution was used. The concentration of the added enzyme was 0.2 or 1.0 U/μL. As the negative control, the reaction was carried out under the condition that the enzyme was not added. By using a thermal cycler, the reaction was conducted at 25°C for 1 hour, and then, the reaction was terminated by heating the reaction solution at 80°C for 5 minutes.

3) Analysis by HPLC and LC-TOF/MS

The reaction solution was analyzed by HPLC using ACQUITY UPLC Oligonucleotide BEH C18 Column (Waters, 2.1 x 100 mm, 1.7 μm). The analysis was conducted with the condition: the column temperature of 80°C, the detection wavelength of 260 nm, the injection amount of 10 μL, and the flow rate of 0.4 mL/min. The linear gradient using the mobile phase of the eluting solution A

(hexafluoroisopropanol-triethylamine) and the eluting solution B (methanol) was used for the analysis. The standards of the sense strand and the antisense strand were similarly analyzed to confirm formation of the ligation products. The mass analyses of the ligation products were carried out by using Agilent 6230 TOF LC/MS System (Agilent Technologies).

4) Results

The analysis results by HPLC are illustrated in FIG. 4. In the HPLC analysis, when the enzyme was added, the peaks of the ligation products were found at the same retention times as the standards, and the peak areas of the modified RNA substrates were decreased as compared with the negative control. In addition, the peak areas of the ligation products increased with the increase of the addition amount of the enzyme. In addition, from the LC-TOF/MS analysis of the reaction solution, formation of the sense strand and the antisense strand were observed under the condition that the enzyme was added.

Sense strand LC/MS m/z: calcd. 2266.13, found 2265.9703 $[M-3H]^{3-}$
Antisense strand LC/MS m/z: calcd. 2531.04, found 2531.0199 $[M-3H]^{3-}$

From the above results, it was found that the siRNA can be formed from four fragments of the modified RNA by the T4 RNA ligase 2.

Example 4: Confirmation of Progress of the Reaction Using DNA Ligase

In the modified oligonucleotide, progress of the enzymatic ligation of the siRNA from the four fragments was evaluated by using a DNA ligase. T4 DNA ligase (New England Biolabs) was used as the DNA ligase.

The reaction solution having the composition of the DNA ligase, 50 mM of Tris-HCl, 10 mM of $MgCl_2$, 10 mM of dithiothreitol, 1 mM of ATP, and pH of 7.5 was used. The concentration of the enzyme was 470 nM; and 10 μM of each oligonucleotide fragment and 30 μL as the volume of the reaction solution were used. The oligonucleotide fragments in the combination listed in Table 3 were used. By using a thermal cycler, the reaction was conducted at 25°C; then, after 4 hours of the reaction, 10 μL of the reaction solution was taken. The reaction was terminated by heating the reaction solution at 80°C for 5 minutes. The concentrations of the ligation products included in the reaction solution were analyzed by HPLC with the condition described in Example 3. The standards were similarly analyzed to quantify the concentrations of the ligation products.

As a result of the HPLC analysis, accumulation of the ligation products was confirmed. After 4 hours of the reaction, accumulations of the sense strand and the antisense strand were 0.58 μM and 5.1 μM, respectively. Accordingly, the formation reaction of the siRNA from the four fragments of the modified RNA was progressed with the DNA ligase as well.

Example 5: Preparation of Deinococcus radiodurans RNA Ligase and Ligation Reaction

(1) Construction of Recombinant Expression Strain by E. coli

The strain capable of expressing the RNA ligase DraRnl in E. coli was constructed, in which the ligase belongs to the Rnl5 family derived from Deinococcus radiodurans; and then, the purified enzyme was prepared. First, a plasmid having the amino acid sequence of DraRnl (SEQ ID NO:17) optimized to E. coli codon was prepared by the gene total synthesis; then, this sequence was sub-cloned to the NdeI/BamHI site of the pET16b vector. This expression plasmid was transformed to E. coli BL21 (DE3) to obtain the expression strain of DraRnl. This expression strain can express the DraRnl provided with His-tag at the N-terminal.

(2) Preparation of Recombinant Enzyme

The expression strains were grown at 37°C overnight in the LB agar medium containing 100 mg/L of ampicillin. The colony thereby obtained was inoculated to 100 mL of the LB medium containing 100 mg/L of ampicillin; then, the shake culture thereof was carried out by using the Sakaguchi flask. After two hours of culturing at 37°C, IPTG and ethanol were added to bring the final concentration thereof to 0.1 mM or 2%, respectively. The culturing was further continued for 16 hours at 17°C.

After completion of culturing, the cells were collected from the obtained culture solution by centrifugal separation, and then, this was suspended in the buffer solution containing 50 mM of Tris-HCl (pH 7.6), 250 mM of NaCl, 10% of sucrose, 15 mM of imidazole, 1% of Lysozyme, and 0.1% of Triton-X100. This suspension was subjected to ultrasonic disintegration, and the cell residues were removed from the disintegrated solution by centrifugal separation to obtain a supernatant as the soluble fraction.

The soluble fraction thus obtained was adsorbed to the supporting body by using the His-tag protein purification column HisTALON Superflow Cartridge (Takara Bio) that had been equilibrated with the above-mentioned buffer solution. The protein not adsorbed to the supporting body (non-absorbable protein) was washed out by the buffer solution containing 50 mM of Tris-HCl (pH 7.6), 250 mM of NaCl, 10% of sucrose, and 15 mM of imidazole; then, the adsorbed protein was eluted out by using the buffer solution containing 50 mM of Tris-HCl (pH 8.0), 250 mM of NaCl, 10% of glycerol, and 200 mM of imidazole.

The eluted fraction including the enzyme was collected; then, the buffer thereof was exchanged with the buffer containing 50 mM of Tris-HCl (pH 8.0), 200 mM of NaCl, 2 mM of DTT, 2 mM of EDTA, 10% of glycerol, and 0.1% of Triton X-10 by using Amicon Ultra-15 10kDa (Merk Millipore) to obtain a purified enzyme solution.

(3) Ligation Reaction by DraRnl

The reaction of four modified RNA fragments was carried out by using DraRnl. The reaction solution having the composition of 50 mM of Tris-HCl (pH 7.5), 10 mM of $MnSO_4$, 1 mM of dithiothreitol, 400 μM of ATP, and 10 μM of each modified RNA fragment, with 25 μL as the volume of the reaction solution was used. The combination of the modified RNA fragments listed in Table 3 was used. The concentration of added enzyme was 72 μg/mL. The reaction was also carried out without addition of the enzyme as the negative control. By using a thermal cycler, the reaction was carried out at 25°C for 3 hours, and then, the reaction was terminated by adding EDTA, the amount of which was to bring the final concentration thereof to 1 mM.

(4) Analysis by HPLC

The reaction solution was analyzed by HPLC using ACQUITY HPLC Oligonucleotide BEH C18 Column (Waters, 2.1 x 100 mm, 1.7 μm). The analysis was conducted with the condition: the column temperature of 60°C, the detection wavelength of 260 nm, the injection amount of 10 μL, and the flow rate of 0.4 mL/min. The linear gradient using the mobile phase of the eluting solution A (hexafluoroisopropanol-triethylamine) and the eluting solution B (methanol) was used for the analysis. The standards of the sense strand and the antisense strand were similarly analyzed to confirm formation of the ligation products.

The analysis results by HPLC are illustrated in FIG. 5. In the HPLC analysis, when the enzyme was added, the peaks of the ligation products were found at the same retention times as the standards, and the peak areas of the modified RNA substrates were decreased as compared with the negative control. Accordingly, it was found that the target modified oligopeptide can be formed from the four modified RNA fragments by DraRnl.

Example 6: Formation of Modified Oligonucleotide Having Loop Structure

Progress of the reaction to form the modified oligonucleotide having a loop structure was evaluated by the enzymatic ligation of four oligonucleotide fragments. The sequences of the target product and of the synthesized substrate fragments are listed in Table 4.

Table 4. Sequence of product and substrate

| Name | Sequence | SEQ ID NO |
|---|---|---|
| Product | C^A(F)U(Me)GAGUGACAAAGCAUUCCCA(F)A(Me)CGGAAUGCUUUGUC ACUCAUGGCUtCG^G | 18 |
| Substrate fragment (1) | C^A(F)U(Me)GAGUGACAA | 19 |
| Substrate fragment (2) | Pho-AGCAUUCCCA(F)A(Me)CGGA | 20 |
| Substrate fragment (3) | Pho-AUGCUUUGUCA | 21 |
| Substrate fragment (4) | Pho-CUCAUGGCUtCG^G | 22 |
| English capital letter: RNA;<br>Pho: modification at 5'-end by phosphate group;<br>(F) : modification by 2'-fluoro group;<br>(Me) : modification by 2'-O-methyl group;<br>^: substitution of phosphate group with thiophosphate group;<br>t: thymidine.<br>The product has the sequence in which the substrate fragments are linked in a direction of 5'-end to 3'-end in the order of (1) to (4) | | |

The reaction was carried out using the four substrate oligonucleotide fragments listed in Table 4 by the T4 RNA ligase 2 (New England Biolabs). The reaction solution having the composition of 50 mM of Tris-HCl, 2 mM of $MgCl_2$, 1 mM of dithiothreitol, 400 μM of ATP, pH of 7.5, and 10 μM of each modified oligonucleotide fragment, with 100 μL as the volume of the reaction solution was used. The added enzyme concentration of 17.8 μg/μL was used to compare the product concentration. As the negative control, the reaction was carried out under the condition that the enzyme was not added. By using a thermal cycler, the reaction was carried out at 25°C for 3 hours, and then, the reaction was terminated by heating the reaction solution at 80°C for 5 minutes. The reaction solution was analyzed by HPLC with the condition of Example 5 and with LC-TOF/MS.

In the HPLC analysis as illustrated in FIG. 6, the peak areas of the modified RNA substrates were decreased as compared with the negative control; on the other hand, the peak could be detected (around 5.6 minutes) at the position where the retention time is longer than the substrates. In addition, from the LC-TOF/MS analysis of the reaction solution, the target product was observed under the condition that the enzyme was added.
LC/MS m/z: calcd. 2727.33, found 2727.21 $[M-6H]^{6-}$

From the above results, it was found that the target modified oligonucleotide having a loop structure can be produced from the four fragments by the T4 RNA ligase 2.

Example 7: Formation of Heteroduplex Composed of DNA Strand and RNA Strand

By using a double strand RNA ligase, the heteroduplex composed of the modified DNA strand and the modified RNA strand was formed. The T4 RNA ligase 2 (New England Biolabs) was used as the double strand RNA ligase.

The reaction solution having the composition of 50 mM of Tris-HCl, 2 mM of $MgCl_2$, 1 mM of dithiothreitol, 400 μM of ATP, and pH of 7.5 was used. The enzyme concentration of 3.56 μg/mL was used. The four oligonucleotide fragments listed in Table 5 were used as the substrates with the concentration of 10 μM each. The reaction solution of 40 μL was used. By using a thermal cycler, the reaction was carried out at 37°C; and after 18 hours of the reaction, the reaction was terminated by adding EDTA, the amount of which was to bring the final concentration thereof to 10 mM. As the negative control, the reaction was carried out under the condition that the enzyme was not added. The concentrations of the ligation products contained in the reaction solution were analyzed by HPLC with the condition of Example 5 and with LC-TOF/MS.

In the HPLC analysis, when the enzyme was added, the peak area of each substrate was decreased as compared with the negative control, and two peaks could be newly detected. In addition, from the LC-TOF/MS analysis of the reaction solution, formation of the modified DNA strand and the modified RNA strand were observed under the condition that the enzyme was added.

Modified DNA strand LC/MS m/z: calcd. 2119.50, found 2119.34 $[M-2H]^{2-}$
Modified RNA strand LC/MS m/z: calcd. 2126.89, found 2126.36 $[M-2H]^{2-}$

From the above results, it was found that the heteroduplex composed of the modified DNA strand and the modified RNA strand can be formed by the T4 RNA ligase 2.

Table 5. Sequence of product and substrate

| Name | Sequence | SEQ ID NO |
|---|---|---|
| Modified type DNA strand product | G(L)mC(L)A(L)mC(L)tggcaG(L)T(L)mC(L)G(L) | 23 |
| Modified type RNA strand product | C(Me)G(Me)A(Me)C(Me)UGC(Me)CA(Me)G(Me)U(Me)G(Me)C(Me) | 24 |
| Modified type DNA strand 5' side fragment | G(L)mC(L)A(L)mC(L)tggc | - |
| Modified type DNA strand 3' side fragment | Pho-aG(L)T(L)mC(L)G(L) | - |
| Modified type RNA strand 5' side fragment | C(Me)G(Me)A(Me)C(Me)UGC(Me)C | - |
| Modified type RNA strand 3' side fragment | Pho-A(Me)G(Me)U(Me)G(Me)C(Me) | - |
| English small letter: DNA;<br>English capital letter: RNA;<br>mC: 5-methyl cytidine;<br>Pho: modification at 5'-endo by phosphate group;<br>(L): locked nucleic acid (LNA);<br>(Me) : modification by 2'-O-methly group. | | |

Example 8: Reaction of Oligonucleotide Fragments Comprising Mismatched Base Pair

Progress of the reaction to form the double strand modified oligonucleotide having a mismatched portion in the base pair from the four oligonucleotide fragments was evaluated by the enzymatic ligation. Each strand of the target product was designated as the strand A or the strand B. The sequences of the target products and of the synthesized substrate fragments are listed in Table 6, and the combination of the four fragments is illustrated in FIG. 7.

The reaction solution having the composition of 1.78 μg/mL of the T4 RNA ligase 2 (New England Biolabs), 50 mM of Tris-HCl, 2 mM of $MgCl_2$, 1 mM of dithiothreitol, 400 μM of ATP, and pH of 7.5 was used. The four oligonucleotide fragments were added as the substrates with the final concentration of 10 μM each, and the reaction was carried out with the solution volume of 40 μL. By using a thermal cycler, the reaction was carried out at 25°C; and after 4 hours of the reaction, the reaction was terminated by adding EDTA, the amount of which was to bring the final concentration thereof to 10 mM. As the negative control, the reaction was carried out under the condition that the enzyme was not added. The concentrations of the ligation products contained in the reaction solution were analyzed by HPLC with the condition of Example 5 and with LC-TOF/MS.

In the HPLC analysis, when the enzyme was added, the peak area of each substrate was decreased as compared with the negative control, and two peaks could be newly detected. In addition, from the LC-TOF/MS analysis of the reaction solution, formation of the strand A and the strand B were confirmed under the condition that the enzyme was added.

Strand A LC/MS m/z: calcd. 1361.77, found 1361.75 $[M-5H]^{5-}$
Strand B LC/MS m/z: calcd. 1517.41, found 1517.35 $[M-5H]^{5-}$

From the above results, it was found that the double strand modified oligonucleotide having the mismatched portion can be formed by the T4 RNA ligase 2.

Table 6. Sequence of product and substrate

| Name | Sequence | SEQ ID NO |
|---|---|---|
| Strand A Product | A(F)^A(Me)^C(F)G(Me)G(F)U(Me)G(F)U(Me)U(F)C(F)U(F)U(Me)G(F)C(Me)U(F)C(Me)U(F)A(Me)U(F)A(Me)A(F) | 25 |
| Strand B Product | U(Me)^U(F)^A(Me)U(F)A(Me)G(F)A(Me)G(F)C(Me)A(F)A(Me)G(Me)A(Me)A(F)C(Me)A(F)C(Me)U(F)G(Me)U(F)U(Me)^U(Me)^U(Me) | 26 |
| Strand A 5' side fragment | A(F)^A(Me)^C(F)G(Me)G(F)U(Me)G(F)U(Me)U(F)C(F)U(F)U(Me) | 27 |
| Strand A 3' side fragment | Pho-G(F)C(Me)U(F)C(Me)U(F)A(Me)U(F)A(Me)A(F) | - |
| Strand B 5' side fragment | U(Me)^U(F)^A(Me)U(F)A(Me)G(F)A(Me)G(F)C(Me)A(F)A(Me)G(Me)A(Me) | 28 |
| Strand B 3' side fragment | Pho-A(F)C(Me)A(F)C(Me)U(F)G(Me)U(F)U(Me)^U(Me)^U(Me) | 29 |
| Pho: modification at 5'-end by phosphate group;<br>English capital letter: RNA;<br>(F) : modification by 2'-fluoro group;<br>(Me) : modification by 2'-O-methyl group;<br>^: thiophosphate linkage. | | |

Example 9: Reaction Using Five and Six Oligonucleotide Fragments

Progress of the reaction to form the double strand modified oligonucleotide from the five and six oligonucleotide fragments was evaluated by the enzymatic ligation. Each strand of the target product was designated as the strand A or the strand B. The sequences of the target products and of the synthesized substrate fragments are listed in Table 7, and the combinations of the four fragments are illustrated in FIG. 8.

The reaction solution having the composition of 1.78 μg/mL of the T4 RNA ligase 2 (New England Biolabs), 50 mM of Tris-HCl, 2 mM of $MgCl_2$, 1 mM of dithiothreitol, 400 μM of ATP, and pH of 7.5 was used. The oligonucleotide fragments were added as the substrates with the final concentration of 10 μM each, and the reaction was carried out with the solution volume of 30 μL. By using a thermal cycler, the reaction was carried out at 37°C; and after 16 hours of the reaction, the reaction was terminated by adding EDTA, the amount of which was to bring the final concentration thereof to 10 mM. As the negative control, the reaction was carried out under the condition that the enzyme was not added. The concentrations of the ligation products contained in the reaction solution were analyzed by HPLC with the condition of Example 5.

As can be illustrated in FIG. 9, in the HPLC analysis, when the enzyme was added, the peak area of each substrate was decreased as compared with the negative control, and two peaks could be newly detected. The retention times of these peaks were the same as those of the product standards.

From the above results, it was found that the double strand modified oligonucleotide can be formed from the five and six oligonucleotide substrates by the T4 RNA ligase 2.

Table 7. Sequence of product and substrate

| Usage | Name | Sequence | SEQ ID NO |
|---|---|---|---|
| Standard | Strand A Product | C (Me) CG (F) CU (F) AGGG (F) U (F) GA (Me) G (Me) CAUA (F) GC (Me) U (Me) G | 30 |
| | Strand B Product | CA (Me) G (F) CU (Me) AU (F) GCU (F) CA (Me) C (Me ) CC (Me) UA (Me) GC (F) G (Me) G | 31 |
| Reaction from 5 fragments | Srand A 5' side fragment | C (Me) CG (F) CU (F) AGGG (F) | |
| | Strand A 3' side fragment | Pho- U (F) GA (Me) G (Me) CAUA (F) GC (Me) U (Me) G | 32 |
| Reaction from 6 fragments | Strand A 5' side fragment | CA (Me) G (F) CU (Me) AU (F) GC | |
| | Strand A central fragment | Pho-U (F) CA (Me) C (Me) CC (Me) U | |
| | Strand A 3' side fragment | Pho-A(Me)GC(F)G(Me)G | |
| Reaction from 5 fragments and 6 fragments | Strand B 5' side fragment | CA (Me) G (F) CU (Me) AU (F) GC | |
| | Strand B central fragment | Pho-U (F) CA (Me) C (Me) CC (Me) U | |
| | Strand B 3' side fragment | Pho-A (Me) GC (F) G (Me) G | |
| Pho: modification at 5'-end by phosphate group;<br>English capital letter: RNA;<br>(F) : modification by 2'-fluoro group;<br>(Me) : modification by 2'-O-methyl group. | | | |

Example 10: Reaction Using Oligonucleotides Having DMTr Group Attached to 5' End

Progress of the reaction to form the double strand modified oligonucleotide from four oligonucleotide fragments comprising two fragments having the dimethoxytrityl (DMTr) group attached to the 5' end thereof was evaluated by the enzymatic ligation. Each strand of the target product was designated as the strand A or the strand B. The sequences of the target products and of the synthesized substrate fragments are listed in Table 8, and the combination of the four fragments is illustrated in FIG. 10.

The reaction solution having the composition of 1.78 μg/mL of the T4 RNA ligase 2 (New England Biolabs), 50 mM of Tris-HCl, 2 mM of $MgCl_2$, 1 mM of dithiothreitol, 400 μM of ATP, and pH of 7.5 was used. Four oligonucleotide fragments were added as the substrates with the final concentration of 10 μM each, and the reaction was carried out with the solution volume of 40 μL. By using a thermal cycler, the reaction was carried out at 25°C; and after 4 hours of the reaction, the reaction was terminated by adding EDTA, the amount of which was to bring the final concentration thereof to 10 mM. As the negative control, the reaction was carried out under the condition that the enzyme was not added. The concentrations of the ligation products contained in the reaction solution were analyzed by HPLC with the condition of Example 5 and with LC-TOF/MS.

In the HPLC analysis, when the enzyme was added, the peak area of each substrate was decreased as compared with the negative control, and two peaks could be newly detected. In addition, from the LC-TOF/MS analysis of the reaction solution, formation of the strand A and the strand B were confirmed under the condition that the enzyme was added.

Strand A LC/MS m/z: calcd. 1764.80, found 1764.79 $[M-4H]^{4-}$
Strand B LC/MS m/z: calcd. 1738.76, found 1738.75 $[M-4H]^{4-}$

From the above results, it was found that the double strand modified oligonucleotide can be formed from the substrate fragments having the DMTr group by the T4 RNA ligase 2.

Table 8. Sequence of product and substrate

| Name | Sequence | SEQ ID NO |
|---|---|---|
| Strand A Product | DMTr-GU(Me)AAC(Me)C(Me)AAGAGU(Me)AU(Me)U(Me)C(Me)C(Me)AU(Me)tt | 33 |
| Strand B Product | DMTr-AUGGAAU(Me)ACUCUUGGUU(Me)ACtt | 34 |
| Strand A 5' side fragment | DMTr-GU(Me)AAC(Me)C(Me)AAGAG | 35 |
| Strand A 3' side fragment | Pho-U(Me)AU(Me)U(Me)C(Me)C(Me)AU(Me)tt | 36 |
| Strand B 5' side fragment | DMTr-AUGGAAU(Me)ACUCU | 37 |
| Strand B 3' side fragment | Pho-UGGUU(Me)ACtt | - |
| Pho: modification at 5'-end by phosphate group;<br>DMTr: modification at 5'-end by DMTr;<br>English capital letter: RNA;<br>(Me): modification by 2'-O-methyl group;<br>t: thymidine residue. | | |

Example 11: Reaction Using Carrier-added Oligonucleotide Fragments

Progress of the reaction to form the double strand modified oligonucleotide from four oligonucleotide fragments comprising one fragment having N-acetylgalactosamine (GalNAc) attached to the 5' end thereof was evaluated by the enzymatic ligation. Each strand of the target product was designated as the strand A or the strand B. The sequences of the target products and of the synthesized substrate fragments are listed in Table 9, and the combination of the four fragments is illustrated in FIG. 11. The GalNAc-modified fragment was synthesized by linking Trivalent β-D-GalNAc with carboxyl-functionalized PEG5 Linker (Sussex Research) at the 5' end of the oligonucleotide via the amino C6 linker.

The reaction solution having the composition of 1.78 μg/mL of the T4 RNA ligase 2 (New England Biolabs), 50 mM of Tris-HCl, 2 mM of $MgCl_2$, 1 mM of dithiothreitol, 400 μM of ATP, and pH of 7.5 was used. Four oligonucleotide fragments were added as the substrates with the final concentration of 10 μM each, and the reaction was carried out with the solution volume of 40 μL. By using a thermal cycler, the reaction was carried out at 25°C; and after 4 hours of the reaction, the reaction was terminated by adding EDTA, the amount of which was to bring the final concentration thereof to 10 mM. As the negative control, the reaction was carried out under the condition that the enzyme was not added. The concentrations of the ligation products contained in the reaction solution were analyzed by HPLC with the condition of Example 5 and with LC-TOF/MS.

In the HPLC analysis, when the enzyme was added, the peak area of each substrate was decreased as compared with the negative control, and two peaks could be newly detected. In addition, from the LC-TOF/MS analysis of the reaction solution, formation of the strand A and the strand B were confirmed under the condition that the enzyme was added.

Strand A LC/MS m/z: calcd. 1730.40, found 1730.37 $[M-5H]^{5-}$
Strand B LC/MS m/z: calcd. 1330.38, found 1330.36 $[M-5H]^{5-}$

From the above results, it was found that the double strand modified oligonucleotide having the end thereof modified with N-acetylgalactosamine can be formed by the reaction from the four fragments using the T4 RNA ligase 2.

Table 9. Sequence of product and substrate

| Name | Sequence | SEQ ID NO |
|---|---|---|
| Strand A Product | GalNAc-GU(Me)AAC(Me)C(Me)AAGAGU(Me)AU(Me)U(Me)C(Me)C(Me)AU(Me)tt | 33 |
| Strand B Product | AUGGAAU (Me) ACUCUUGGUU (Me) ACtt | 34 |

(continued)

| Name | Sequence | SEQ ID NO |
|---|---|---|
| Strand A 5' side fragment | GalNAc-GU(Me)AAC(Me)C(Me)AAGAG | 35 |
| Strand A 3' side fragment | Pho-U(Me)AU(Me)U(Me)C(Me)C(Me)AU(Me)tt | 36 |
| Strand B 5' side fragment | AUGGAAU(Me)ACUCU | 37 |
| Strand B 3' side fragment | Pho-UGGUU(Me)ACtt | |
| Pho: modification at 5'-end by phosphate group;<br>GalNAc: modification at 5'-end by N-acetylgalactosamine;<br>English capital letter: RNA;<br>(Me): modification by 2'-O-methyl group;<br>t: thymidine residue. | | |

Example 12: Formation Reaction of Double Strand Modified Oligonucleotide Having Thiophosphate Diester linkage at the Linking Site

Progress of the reaction to form the double strand modified oligonucleotide from the four oligonucleotide fragments having the phosphate group thereof displaced with a thiophosphate group was evaluated by the enzymatic ligation. Each strand of the target product was designated as the strand A or the strand B. The sequences of the target products and of the synthesized substrate fragments are listed in Table 10, and the combination of the four fragments is illustrated in FIG. 12.

The reaction solution having the composition of 1.78 μg/mL of the T4 RNA ligase 2 (New England Biolabs), 50 mM of Tris-HCl, 2 mM of $MgCl_2$, 1 mM of dithiothreitol, 400 μM of ATP, and pH of 7.5 was used. Four oligonucleotide fragments were added as the substrates with the final concentration of 10 μM each, and the reaction was carried out with the solution volume of 40 μL. By using a thermal cycler, the reaction was carried out at 25°C; and after 4 hours of the reaction, the reaction was terminated by adding EDTA, the amount of which was to bring the final concentration thereof to 10 mM. As the negative control, the reaction was carried out under the condition that the enzyme was not added. The concentrations of the ligation products contained in the reaction solution were analyzed by HPLC with the condition of Example 5 and with LC-TOF/MS.

In the HPLC analysis, when the enzyme was added, the peak area of each substrate was decreased as compared with the negative control, and two peaks could be newly detected. In addition, from the LC-TOF/MS analysis of the reaction solution, under the condition that the enzyme was added, the strand A and the strand B were detected, and thus, formation thereof could be confirmed.

Strand A LC/MS m/z: calcd. 1769.15, found 1769.13 $[M-4H]^{4-}$
Strand B LC/MS m/z: calcd. 1743.11, found 1743.10 $[M-4H]^{4-}$

From the above results, it was found that the double strand modified oligonucleotide having the thiophosphate linkage at the linking site can be formed by the reaction from the four fragments using the T4 RNA ligase 2.

Table 10. Sequence of product and substrate

| Name | Sequence | SEQ ID NO |
|---|---|---|
| Strand A Product | G^U(Me)^A^A^C(Me)^C(Me)^A^A^G^A^G^ U(Me)^A^U(Me)^U(Me)^C(Me)^C(Me)^A^U(Me)^t^t | 33 |
| Strand B Product | A^U^G^G^A^A^U(Me)^A^C^U^C^U^ U^G^G^U^U(Me)^A^C^t^t | 34 |
| Strand A 5' side fragment | G^U(Me)^A^A^C(Me)^C(Me)^A^A^G^A^G | 35 |
| Strand A 3' side fragment | PS- U(Me)^A^U(Me)^U(Me)^C(Me)^C(Me)^A^U(Me)^t^t | 36 |
| Strand B 5' side fragment | A^U^G^G^A^A^U(Me) ^A^C^U^C^U | 37 |

(continued)

| Name | Sequence | SEQ ID NO |
|---|---|---|
| Strand B 3' side fragment | PS-U^G^G^U^U(Me) ^A^C^t^t | - |
| PS: modification at 5'-end by thiophosphate group;<br>English capital letter: RNA;<br>^: thiophosphate linkage;<br>(Me): modification by 2'-O-methyl group;<br>t: thymidine residue. | | |

Example 13: Formation Reaction of Hairpin Type Oligonucleotide

Progress of the reaction to form the hairpin type oligonucleotide from the four oligonucleotide fragments was evaluated by the enzymatic ligation. The sequences of the target product and of the synthesized substrate fragments are listed in Table 11, and the combination of the four fragments is illustrated in FIG. 13. The proline derivative described in the literature (Hamasaki T., Suzuki H., Shirohzu H., et al., Efficacy of a novel class of RNA interference therapeutic agents. PLoS ONE. 2012; 7(8): e42655) was used as the linker.

The reaction solution having the composition of 1.78 μg/mL of the T4 RNA ligase 2 (New England Biolabs), 50 mM of Tris-HCl, 2 mM of $MgCl_2$, 1 mM of dithiothreitol, 400 μM of ATP, and pH of 7.5 was used. Four oligonucleotide fragments were added as the substrates with the final concentration of 10 μM each, and the reaction was carried out with the solution volume of 40 μL. By using a thermal cycler, the reaction was carried out at 25°C; and after 4 hours of the reaction, the reaction was terminated by adding EDTA, the amount of which was to bring the final concentration thereof to 10 mM. As the negative control, the reaction was carried out under the condition that the enzyme was not added. The concentration of the ligation product contained in the reaction solution was analyzed by HPLC with the condition of Example 5 and with LC-TOF/MS.

In the HPLC analysis, when the enzyme was added, the peak area of each substrate was decreased as compared with the negative control, and one peak could be newly detected. In addition, from the LC-TOF/MS analysis of the reaction solution, under the condition that the enzyme was added, the target product was detected, and thus, formation thereof could be confirmed.

LC/MS m/z: calcd. 1891.61, found 1891.60 $[M-9H]^{9-}$

From the above results, it was found that the hairpin type oligonucleotide can be formed by the reaction from the four fragments using the T4 RNA ligase 2.

Table 11. Sequence of product and substrate

| Name | Sequence | SEQ ID NO |
|---|---|---|
| Product | AGCAGAGUACACACAGCAUAUACC-Pro-<br>GGUAUAUGCUGUGUGUACUCUGCUUC-Pro-G | 38 |
| Fragment 1 | AGCAGAGUACACAC | 39 |
| Fragment 2 | Pho-AGCAUAUACC-Pro-GGUA | 40 |
| Fragment 3 | Pho-UAUGCUGUGUGU | 41 |
| Fragment 4 | Pho-ACUCUGCUUC-Pro-G | 42 |
| Pho: modification at 5'-end by phosphate group;<br>English capital letter: RNA<br>Pro: proline derivative. | | |

Example 14: Impact of Nucleotide Length in Sticky end on Reactivity

Oligonucleic acid substrates were designed to form the same product and have the sticky portions with the nucleotide length of 1 to 6; then, the difference in the reactivity thereof due to the difference in the nucleotide length of the sticky portion was compared. Each strand of the target product was designated as the strand A or the strand B. In all the combinations, the sequences of the substrates that constitute the strand B were made same, while the cleaving site in the strand A was changed. The sequences of the synthesized target product standards and substrate fragments are listed in Table 12, and the combinations of the four fragments are illustrated in FIG. 14.

The reaction solution having the composition of 1.78 μg/mL of the T4 RNA ligase 2 (New England Biolabs), 50 mM of Tris-HCl, 2 mM of $MgCl_2$, 1 mM of dithiothreitol, 400 μM of ATP, and pH of 7.5 was used. Four oligonucleotide fragments were added as the substrates with the final concentration of 10 μM each, and the reaction was carried out with the solution volume of 40 μL. By using a thermal cycler, the reaction was carried out at 25°C; and after 15 minutes, 30 minutes, 1 hour, 2 hours, and 4 hours of the reaction, 5 μL each of the reaction solution was collected, and each reaction thereof was terminated by adding EDTA, the amount of which was to bring the final concentration thereof to 10 mM. As the negative control, the reaction was carried out under the condition that the enzyme was not added. The concentrations of the ligation products contained in the reaction solution and of the product standards were analyzed by HPLC with the condition of Example 5 to calculate the concentrations of the ligation products.

As a result of confirmation with the HPLC analysis, with all the nucleotide lengths in the sticky portions, formation of the strand A and the strand B were observed. There was a tendency that the reaction rate was slow when the sticky portion had one nucleotide length.

Table 12. Sequence of product and substrate

| Usage | Name | Sequence | SEQ ID NO |
|---|---|---|---|
| Standard | Strand A Product | GC(Me)U(Me)AAC(Me)C(Me)GGGGGGGG U(Me)GC(Me)C(Me)AG(Me) | 43 |
| | Strand B Product | CUGGC(Me)AC(Me)C(Me)C(Me)C(Me)C (Me)C(Me)C(Me)C(Me)GGUU(Me)AGC( Me) | 44 |
| Reaction of sticky end having the following nucleotide length | | | |
| 6 nt | Strand A 5' side fragment | GC(Me)U(Me)AAC(Me)C(Me)GGGGGGG | 45 |
| | Strand A 3' side fragment | Pho-GU(Me)GC(Me)C(Me)AG(Me) | - |
| 5 nt | Strand A 5' side fragment | GC(Me)U(Me)AAC(Me)C(Me)GGGGGG | 46 |
| | Strand A 3' side fragment | Pho-GGU(Me)GC(Me)C(Me)AG(Me) | - |
| 4 nt | Strand A 5' side fragment | GC(Me)U(Me)AAC(Me)C(Me)GGGGG | 47 |
| | Strand A 3' side fragment | Pho-GGGU(Me)GC(Me)C(Me)AG(Me) | - |
| 3 nt | Strand A 5' side fragment | GC(Me)U(Me)AAC(Me)C(Me)GGGG | 48 |
| | Strand A 3' side fragment | Pho-GGGGU(Me)GC(Me)C(Me)AG(Me) | 49 |
| 2 nt | Strand A 5' side fragment | GC(Me)U(Me)AAC(Me)C(Me)GGG | 50 |
| | Strand A 3' side fragment | Pho-GGGGGU(Me)GC(Me)C(Me)AG(Me) | 51 |
| 1 nt | Strand A 5' side fragment | GC(Me)U(Me)AAC(Me)C(Me)GG | - |
| | Strand A 3' side fragment | Pho- GGGGGGU(Me)GC(Me)C(Me)AG(Me) | 52 |
| Substrate fragments common to each reaction | Strand B 5' side fragment | CUGGC(Me)AC(Me)C(Me)C(Me)C(Me)C (Me)C(Me)C(Me) | 53 |
| | Strand B 3' side fragment | Pho-C(Me)GGUU(Me)AGC(Me) | - |
| Pho: modification at 5'-end by phosphate group;<br>English capital letter: RNA;<br>(Me): modification by 2'-O-methyl group. | | | |

Example 15: Impact of Nucleotide Length in Product on Reactivity

The difference in the reactivity was compared when a short target product having the complementary portion with the nucleotide length of 11 to 14 was produced. Each strand of the target product was designated as the strand A or the strand B. The sequences of the target products and of the synthesized substrate fragments are listed in Table 13, and the combinations of the four fragments are illustrated in FIG. 15.

The reaction solution having the composition of 1.78 μg/mL of the T4 RNA ligase 2 (New England Biolabs), 50 mM of Tris-HCl, 2 mM of $MgCl_2$, 1 mM of dithiothreitol, 400 μM of ATP, and pH of 7.5 was used. Four oligonucleotide fragments were added as the substrates with the final concentration of 10 μM each, and the reaction was carried out with the solution volume of 40 μL. By using a thermal cycler, the reaction was carried out at 25°C; and after 4 hours of the reaction, the reaction was terminated by adding EDTA, the amount of which was to bring the final concentration thereof to 10 mM. As the negative control, the reaction was carried out under the condition that the enzyme was not added. The concentrations of the ligation products contained in the reaction solution were analyzed by HPLC with the condition of Example 5 and with LC-TOF/MS.

In the HPLC analysis, when the enzyme was added, the peak area of each substrate was decreased as compared with the negative control, and a new peak could be detected at the position in a later retention time. In addition, from the LC-TOF/MS analysis of the reaction solution, under the condition that the enzyme was added, the divalent or trivalent ions of the strand A and strand B were detected in products of all the nucleotide lengths, as listed in Table 14; thus, progress of the reaction could be confirmed.

The peak areas of substrates under the respective conditions in HPLC analysis were calculated by the following formula to obtain the residual rate of the substrates.

Residual rate (%) = (total peak area of substrates with enzyme addition)/total peak area of substrates in negative control) x 100

Then, the tendency was found that the residual rate of the substrates in the product having the complementary portion with the nucleotide length of 11 was higher than the residual rate of the substrates in the product having the complementary portion with the nucleotide length of 12 or more.

Table 13. Sequence of product and substrate

| Nucleotide length of product | Name | Sequence | SEQ ID NO |
|---|---|---|---|
| 14 nt | Strand A Product | G(Me)CC(Me)C(Me)AC(Me)GAU(Me)AGU(Me)GC(Me) | 54 |
| | Strand B Product | GC(Me)AC(Me)U(Me)AU(Me)CGU(Me)GGG(Me)C | 55 |
| | Strand A 5' side fragment | G(Me)CC(Me)C(Me)AC(Me)GA | - |
| | Strand A 3' side fragment | U(Me)AGU(Me)GC(Me) | - |
| | Strand B 5' side fragment | GC(Me)AC(Me)U(Me)AU(Me)CG | - |
| | Strand B 3' side fragment | U (Me) GGG (Me) C | - |
| 13 nt | Strand A Product | G(Me)CC(Me)C(Me)AC(Me)GAU(Me)GU(Me)GC(Me) | 56 |
| | Strand B Product | GC(Me)AC(Me)AU(Me)CGU(Me)GGG(Me)C | 57 |
| | Strand A 5' side fragment | The same as Strand A, 5' side fragment in the product (14 nt) | |
| | Strand A 3' side fragment | U(Me)GU(Me)GC(Me) | - |
| | Strand B 5' side fragment | GC(Me)AC(Me)AU(Me)CG | - |
| | Strand B 3' side fragment | The same as Strand B, 3' side fragment in the product (14 nt) | |
| 12 nt | Strand A Product | G (Me) CC (Me) C (Me) ACGU (Me) GU (Me) GC (Me) | 58 |
| | Strand B Product | GC(Me)AC(Me)ACGU(Me)GGG(Me)C | 59 |
| | Strand A 5' side fragment | G(Me)CC(Me)C(Me)ACG | - |
| | Strand A 3' side fragment | U(Me)GU(Me)GC(Me) | - |
| | Strand B 5' side fragment | GC (Me) AC (Me) ACG | - |
| | Strand B 3' side fragment | U (Me) GGG (Me) C | - |

(continued)

| Nucleotide length of product | Name | Sequence | SEQ ID NO |
|---|---|---|---|
| 11 nt | Strand A Product | G(Me)CC(Me)C(Me)ACGU(Me)GU(Me)G | 60 |
| | Strand B Product | C(Me)AC(Me)ACGU(Me)GGG(Me)C | 61 |
| | Strand A 5' side fragment | The same as Strand A, 5' side fragment in the product (14 nt) | |
| | Strand A 3' side fragment | U (Me) GU (Me) G | - |
| | Strand B 5' side fragment | C (Me) AC (Me) ACG | - |
| | Strand B 3' side fragment | The same as Strand B, 3' side fragment in the product (14 nt) | |
| Pho: modification at 5'-end by phosphate group;<br>English capital letter: RNA<br>(Me): modification by 2'-O-methyl group. | | | |

Table 14. Confirmation of each product by mass spectrometry

| Nucleotide length of product | Strand A | | Strand B | |
|---|---|---|---|---|
| | m/z calcd | m/z fond | m/z calcd | m/z fond |
| 14 nt | 2269.37 | 2269.36 ($[M-3H]^{3-}$) | 2270.44 | 2269.58 ($[M-3H]^{3-}$) |
| 13 nt | 2104.85 | 2104.84 ($[M-3H]^{3-}$) | 2110.83 | 2110.82 ($[M-3H]^{3-}$) |
| 12 nt | 1933.31 | 1933.31 ($[M-2H]^{2-}$) | 1950.81 | 1950.80 ($[M-2H]^{2-}$) |
| 11 nt | 1773.78 | 1773.77 ($[M-2H]^{2-}$) | 1778.29 | 1778.27 ($[M-2H]^{2-}$) |

Example 16: Reaction with High Substrate Concentrations

The reaction rate of producing the modified oligonucleotide in the presence of higher substrate concentration was compared at different pH. Each strand of the target reaction product was designated as the strand A or the strand B. The sequences of the target products and of the synthesized substrate fragments are listed in Table 15, and the combinations of the four fragments are illustrated in FIG. 16.

The reaction solution having the composition of 1.78 μg/mL of the T4 RNA ligase 2 (New England Biolabs), 2 mM of $MgCl_2$, 1 mM of dithiothreitol, 400 μM of ATP, and 50 mM of Tris-HCl as the buffer (pH of 7.0 to 9.0) was used. Oligonucleotide fragments were added as the substrates with the final concentration of 10 μM, 300 μM, 500 μM, or 1,000 μM each, and the reaction was carried out with the solution volume of 40 μL. By using a thermal cycler, the reaction was carried out at 25°C; and after 15 minutes or 1 hour of the reaction, the reaction was terminated by adding EDTA, the amount of which was to bring the final concentration thereof to 10 mM. The ligation products contained in the reaction solution were analyzed by HPLC with the condition of Example 5; from the total concentration of the strand A and the strand B, the product formation rate was calculated. Progress of the reaction was confirmed with the substrate concentrations of 300 μM or more, too; and under these concentrations, high reaction rates were obtained at pH 8.0 and 8.5.

Table 15. Sequence of product and substrate

| Name | Sequence | SEQ ID NO |
|---|---|---|
| Strand A Product | GU(Me)AAC(Me)C(Me)AAGAGU(Me)AU(Me)U(Me)C(Me)C(Me)AU(Me)tt | 33 |
| Strand B Product | AUGGAAU(Me)ACUCUUGGUU(Me)ACtt | 34 |
| Strand A 5' side fragment | GU(Me)AAC(Me)C(Me)AAGAG | 35 |
| Strand A 3' side fragment | Pho-U(Me)AU(Me)U(Me)C(Me)C(Me)AU(Me)tt | 36 |
| Strand B 5' side fragment | AUGGAAU(Me)ACUCU | 37 |

(continued)

| Name | Sequence | SEQ ID NO |
|---|---|---|
| Strand B 3' side fragment | Pho-UGGUU(Me)ACtt | - |
| Pho: modification at 5'-end by phosphate group;<br>English capital letter: RNA<br>(Me): modification by 2'-O-methyl group;<br>t: thymidine residue. | | |

Example 17: Impact of Addition of Surfactant

The reaction rate of the production of the modified oligonucleotide by addition of a surfactant was evaluated. The sequences of the target products and of the synthesized substrate fragments are listed in Table 15, and the combination of the four fragments is illustrated in FIG. 16.

The reaction solution having the composition of 1.78 μg/mL of the T4 RNA ligase 2 (New England Biolabs), 2 mM of $MgCl_2$, 1 mM of dithiothreitol, 400 μM of ATP, and 50 mM of Tris-HCl (pH of 7.5) was used. Triton X-100 with the final concentration of 0.1% was used as the surfactant. Oligonucleotide fragments were added as the substrates with the final concentration of 20 μM each; the reaction was carried out with the solution volume of 40 μL.

In the evaluation, the following three conditions were compared: the enzyme solution was diluted to 17.8 μg/mL by a storage buffer (10 mM of Tris-HCl, 50 mM of KCl, 35 mM of ammonium sulfate, 0.1 mM of dithiothreitol, 0.1 mM of EDTA, 50% of glycerol, and pH 7.5), and then, 1/10 of this solution was added to the reaction solution (control condition); the enzyme solution was diluted to 17.8 μg/mL by the storage buffer, and then, 1/10 of this solution was added to the reaction solution containing Triton X-100 with the final concentration 0.1% (test condition 1); the enzyme solution was diluted to 17.8 μg/mL by the storage buffer containing 0.1% Triton X-100, and then, 1/10 of this solution was added to the reaction solution containing 0.09% of Triton X-100 (test condition 2: Triton X-100 with the final concentration of 0.1%).

By using a thermal cycler, the reaction was carried out at 25°C; and after 4 hours of the reaction, the reaction was terminated by adding EDTA, the amount of which was to bring the final concentration thereof to 10 mM. The ligation products contained in the reaction solution were analyzed by HPLC with the condition of Example 5 to calculate the concentrations of the strand A and the strand B.

In the test conditions 1 and 2, higher concentrations of the strand A and strand B products were observed as compared with the control condition.

Example 18: Comparison of Elimination of Impurities due to Difference in Product's Nucleotide Length

The reactions of the substrates and products having different nucleotide lengths were carried out, and then, impurities included in the substrate oligonucleic acid fragments and in the solution after the enzymatic reaction were analyzed. Each strand of the target reaction product was designated as the strand A or the strand B. The sequences of the target products and of the synthesized substrate fragments are listed in Table 16, and the combinations of the four substrate fragments are illustrated in FIG. 17.

In the reaction using the modified oligonucleotide, the reaction solution having the composition of 8.9 μg/mL of the T4 RNA ligase 2 (New England Biolabs), 50 mM of Tris-HCl, 2 mM of $MgCl_2$, 1 mM of dithiothreitol, 400 μM of ATP, and pH of 7.5 was used. In the reaction using the DNA, the composition of 22.0 μg/mL of the T7 DNA ligase (New England Biolabs), 50 mM of Tris-HCl, 2 mM of $MgCl_2$, 1 mM of dithiothreitol, 400 μM of ATP, and pH of 7.5 was used. Four oligonucleotide fragments were added as the substrates with the final concentration of 50 μM each, and the reaction was carried out with the solution volume of 30 μL. By using a thermal cycler, the reaction was carried out at 25°C; and after 8 hours of the reaction, the reaction was terminated by adding EDTA, the amount of which was to bring the final concentration thereof to 12.5 mM. The reaction solution was analyzed by HPLC with the condition of Example 5 and with LC-TOF/MS; then, the progress of the reaction was confirmed.

In accordance with the method of the prior literature (Roussis et al., Journal of Chromatogr A. 2019; 1584: 106 to 114), the content ratio of the impurities (N±1mer) to the product having the target structure was calculated from the obtained mass analysis result. Similarly, the substrate oligonucleotides solution used in the reaction was analyzed by LC-TOF/MS to calculate the content ratio of the impurities (N±1mer) to the substrates.

From the values calculated above, the residual rates (%) of the impurities in the strand A and the strand B were calculated by the following formula.

Residual rate of impurities (%) = (ratio of N±1mer to target product in reaction solution)/(ratio of N±1mer to substrates in substrate solution)x100

The results are listed in Table 17. In the reactions to produce the oligonucleic acids having 28 nucleotide length, the residual rates of the impurities (N±1mer) were 86% or more; on the other hand, in the reactions to produce the oligonucleic acids having 25 or less nucleotide length, the residual rates were 23 to 59%.

Table 16. Sequence of product and substrate

| Product | Name | Sequence | SEQ ID NO |
|---|---|---|---|
| Double strand DNA (28 nt) | Strand A Product | gggtaatgaattccaaagcttcccccta | 62 |
| | Strand B Product | tagggggaagctttggaattcattaccc | 63 |
| | Strand A 5' side fragment | gggtaatga | - |
| | Strand A 3' side fragment | Pho-attccaaagcttcccccta | 64 |
| | Strand B 5' side fragment | tagggggaagctttgg | 65 |
| | Strand B 3' side fragment | Pho-aattcattaccc | 66 |
| Double strand modified oligonucleic acid (28 nt) | Strand A Product | G(Me)GG(Me)UAAUG(Me)AAU(Me)UC(Me)CAA(Me)A(Me)GCUUC(Me)CCCC(Me)UA(Me) | 67 |
| | Strand B Product | UA(Me)GGG(Me)GGA(Me)AG(Me)CUUUG(Me)GAAUUCA(Me)UU(Me)ACC(Me)C | 68 |
| | Strand A 5' side fragment | G(Me)GG(Me)UAAUG(Me)A | - |
| | Strand A 3' side fragment | Pho-AU(Me)UC(Me)CAA(Me)A(Me)GCUUC(Me)CCCC(Me)UA(Me) | 69 |
| | Strand B 5' side fragment | UA(Me)GGG(Me)GGA(Me)AG(Me)CUJUG(Me)G | 70 |
| | Strand B 3' side fragment | Pho-AAUUCA(Me)UU(Me)ACC(Me)C | 71 |
| Double strand modified oligonucleic acid (25 nt) | Strand A Product | G(Me)GG(Me)UAAUG(Me)AAU(Me)UC(Me)CAA(Me)A(Me)GCUUC(Me)CCC | 72 |
| | Strand B Product | GG(Me)GGA(Me)AG(Me)CUUUG(Me)GUA(Me)GGG(Me)GGA(Me)AG(Me)CUUUG(Me)G | 73 |
| | Strand A 5' side fragment | The same as Strand A, 5' side fragment of double strand modified oligonucleic acid (28 nt) | - |
| | Strand A 3' side fragment | Pho-AU(Me)UC(Me)CAA(Me)A(Me)GCUUC(Me)CCC | 74 |
| | Strand B 5' side fragment | GG(Me)GGA(Me)AG(Me)CUUUG(Me)G | 75 |
| | Strand B 3' side fragment | The same as Strand B, 3' side fragment of double strand modified oligonucleic acid (28 nt) | - |

(continued)

| Product | Name | Sequence | SEQ ID NO |
|---|---|---|---|
| Double strand modified oligonucleic acid (23 nt) | Strand A Product | G(Me)GG(Me)UAAUG(Me)AAU(Me)UC(Me)CAA(Me)A(Me)GCUUC(Me)C | 76 |
| | Strand B Product | GGA(Me)AG(Me)CUUUG(Me)GUA(Me)GGG(Me)GGA(Me)AG(Me)CUUUG(Me)G | 77 |
| | Strand A 5' side fragment | The same as Strand A, 5' side fragment of double strand modified oligonucleic acid (28 nt) | - |
| | Strand A 3' side fragment | Pho-AU(Me)UC(Me)CAA(Me)A(Me)GCUUC(Me)C | 78 |
| | Strand B 5' side fragment | GGA(Me)AG(Me)CUUUG(Me)G | 79 |
| | Strand B 3' side fragment | The same as Strand B, 3' side fragment of double strand modified oligonucleic acid (28 nt) | |
| Double strand modified oligonucleic acid (21 nt) | Strand A Product | G(Me)GG(Me)UAAUG(Me)AAU(Me)UC(Me)CAA(Me)A(Me)GCUU | 80 |
| | Strand B Product | A(Me)AG(Me)CUUUG(Me)GAAUUCA(Me)UU(Me)ACC(Me)C | 81 |
| | Strand A 5' side fragment | The same as Strand A, 5' side fragment of double strand modified oligonucleic acid (28 nt) | - |
| | Strand A 3' side fragment | Pho-AU(Me)UC(Me)CAA(Me)A(Me)GCUU | 82 |
| | Strand B 5' side fragment | A(Me)AG(Me)CUUUG(Me)G | - |
| | Strand B 3' side fragment | The same as Strand B, 3' side fragment of double strand modified oligonucleic acid (28 nt) | - |
| Pho: modification at 5'-end by phosphate group;<br>English small letter: DNA;<br>English capital letter: RNA;<br>(Me): modification by 2'-O-methyl group. | | | |

Table 17. Residual rate of impurities (N±1mer)

| | Nucleotide length of modified oligonucleotide | | | | Nucleotide length of DNA |
|---|---|---|---|---|---|
| | 21 | 23 | 25 | 28 | 28 |
| Strand A | 51% | 41% | 50% | 87% | 88% |
| Strand B | 34% | 24% | 59% | 96% | 100% |

INDUSTRIAL APPLICABILITY

The present invention is useful for production of modified oligonucleotides (for example, an siRNA and a heteroduplex oligonucleotide) that can be used for production of a nucleic acid drug and the like.

[SEQUENCE LISTING]

**Claims**

1. A method for producing a modified oligonucleotide comprising a complementary portion having 11 to 27 nucleotide length, the method comprising forming the modified oligonucleotide by treating four or more oligonucleotide raw material fragments in total in the presence of an oligonucleotide ligase,
the four or more oligonucleotide raw material fragments in total correspond to oligonucleotide raw material fragments that are obtained by dividing the modified oligonucleotide at a fragment linking site that satisfies the following conditions (i) to (v):

   (i) one or more fragment linking sites are present in the complementary portion in each strand side, and two or more fragment linking sites in total are present in the modified oligonucleotide;
   (ii) when the modified oligonucleotide is divided at the fragment linking site, a sticky end is formed in the complementary portion, in which the sticky end has 1 to 10 nucleotide length;
   (iii) at least one oligonucleotide raw material fragment has a modified nucleotide;
   (iv) four oligonucleotide raw material fragments out of the four or more oligonucleotide raw material fragments in total include the complementary portion having 5 to 25 nucleotide length; and
   (v) total nucleotide length of the oligonucleotide raw material fragments corresponding to the complementary portion in each strand side is 11 to 27.

2. The method according to claim 1, wherein the sticky end in (ii) has 2 to 6 nucleotide length.

3. The method according to claim 1 or 2, wherein a portion other than the sticky end in the complementary portion of the four oligonucleotide raw material fragments defined by (iv) has 4 to 16 nucleotide length.

4. The method according to any one of claims 1 to 3, wherein the oligonucleotide ligase is an RNA ligase.

5. The method according to claim 4, wherein the oligonucleotide ligase is a double strand RNA ligase.

6. The method according to claim 5, wherein the double strand RNA ligase is an RNA ligase belonging to the Rnl2 family or the Rnl5 family.

7. The method according to any one of claims 1 to 6, wherein the modified oligonucleotide comprises a modified nucleotide residue.

8. The method according to claim 7, wherein the modified nucleotide residue is a 1', 2', 3', or 4' chemically modified type nucleotide residue, a 5'- or 3'-phosphate-modified type nucleotide residue, a bridging type modified nucleotide residue, a carrier-addition type modified nucleotide residue, or a sugar skeleton-displacement type nucleotide residue.

9. The method according to claim 8, wherein the modified nucleotide residue is:

   i) the 1', 2', 3', or 4' chemically modified type nucleotide residue having 1', 2', 3', or 4' position thereof displaced with $C_{1-6}$ alkyloxy $C_{1-6}$ alkylene, -O-$C_{1-6}$ alkyl, -O-$C_{6-14}$ aryl, -C-aryl, a halogen atom, -O-$C_{1-6}$ alkyl N-amide $C_{1-6}$ alkylene, -O-$C_{1-6}$ alkyl-($C_{1-6}$ alkyl-)amino-$C_{1-6}$ alkylene, or -O-amino $C_{1-6}$ alkyl (for example, -O-aminopropyl: -O-AP);
   ii) the 5'- or 3'-phosphate-modified type nucleotide residue displaced with -O-P(S)$(OH)_2$, -NH-P(O)$(OH)_2$, or -NH-P(S) $(OH)_2$, in which the hydroxide group is optionally displaced with a protection group;
   iii) the bridging type modified nucleotide residue having 2' and 4' positions thereof displaced with 2'-O-$C_{1-6}$ alkylene-4', 2'-O-ethylene-4', 2'-O-methyl-substituted methylene-4', 2'-O-$C_{1-6}$ alkylene-O-$C_{1-6}$ alkylene-4', 2'-ON(R)-$C_{1-6}$ alkylene-4' wherein R represents a methyl group, a hydrogen atom, or a benzyl group, 2'-N(R)-C(O)-4', 2'-NH-$C_{1-6}$ alkylene-4', or 2'-$C_{1-6}$ alkylene-4', or a 3' position and a 5' position thereof displaced with 3'-$C_{1-6}$ alkylene-5'; or
   iv) a hexitol nucleic acid (HNA) residue, a cyclohexenyl nucleic acid (CeNA) residue, or a morpholino nucleic acid (PMO) residue.

10. The method according to any one of claims 1 to 9, wherein a total mole ratio of two oligonucleotide raw material fragments arbitrary selected from the oligonucleotide raw material fragments is in a range of 0.5 to 2.

**11.** The method according to any one of claims 1 to 10, wherein the oligonucleotide raw material fragments are subjected to a treatment with a monovalent cationic salt whose concentration is 10 mM or less.

**12.** The method according to any one of claims 1 to 11, wherein prior to the treatment, a mixed solution of the oligonucleotide raw material fragments is not placed at high temperature and is not cooled down thereafter.

**13.** The method according to any one of claims 1 to 12, wherein formation of impurities of the modified oligonucleotide is suppressed.

**14.** The method according to any one of claims 1 to 13, wherein the method further comprises purifying the modified oligonucleotide.

FIG. 1

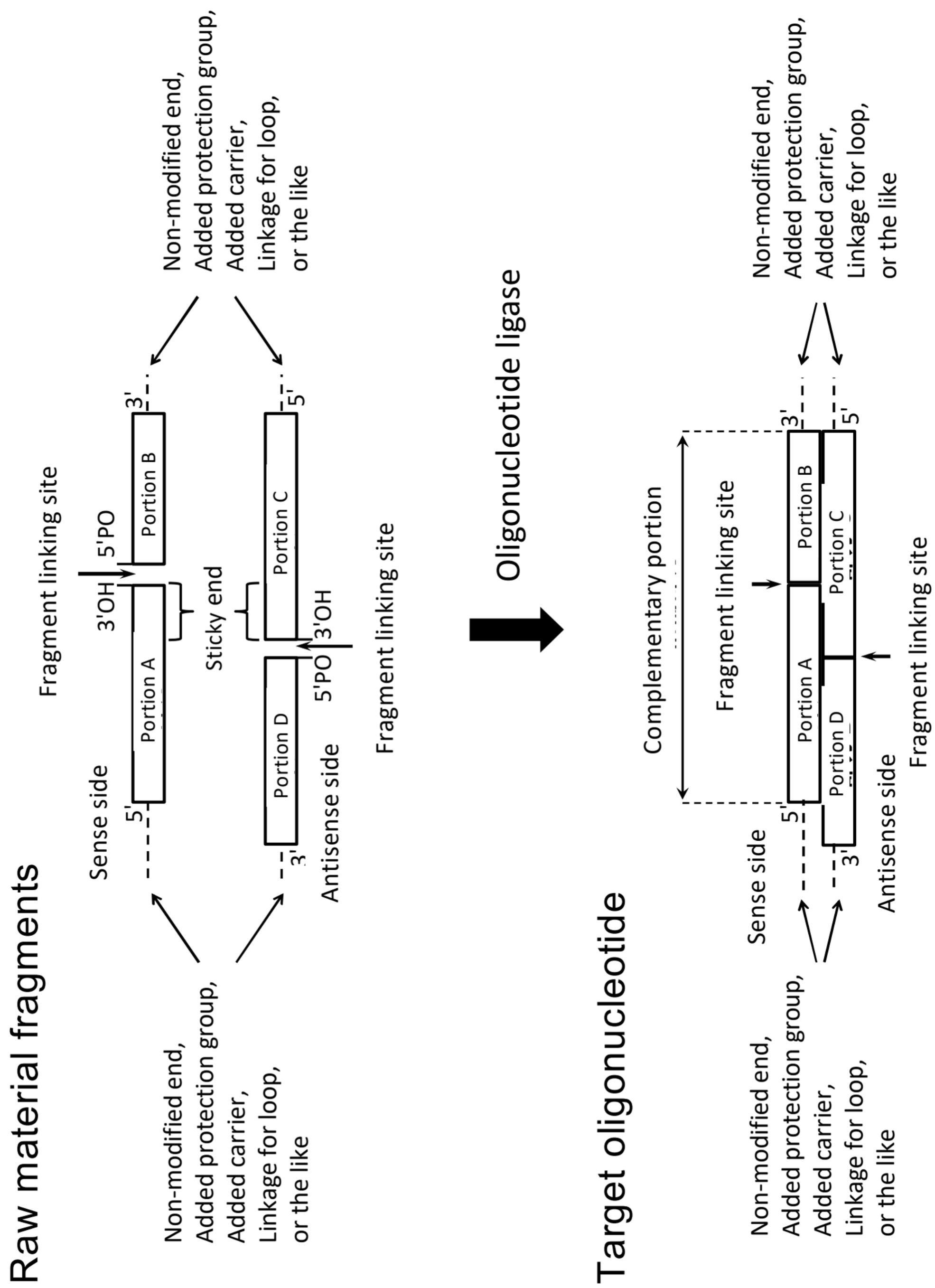

Raw material fragments
Non-modified end,
Added protection group,
Added carrier,
Linkage for loop,
or the like
Sense side
Antisense side
5'
3'
Portion A
Portion B
Portion C
Portion D
3'OH
5'PO
Fragment linking site
Sticky end
Non-modified end,
Added protection group,
Added carrier,
Linkage for loop,
or the like
Oligonucleotide ligase
Target oligonucleotide
Complementary portion
Fragment linking site
Sense side
Antisense side
Portion A
Portion B
Portion C
Portion D
5'
3'

FIG. 2-1

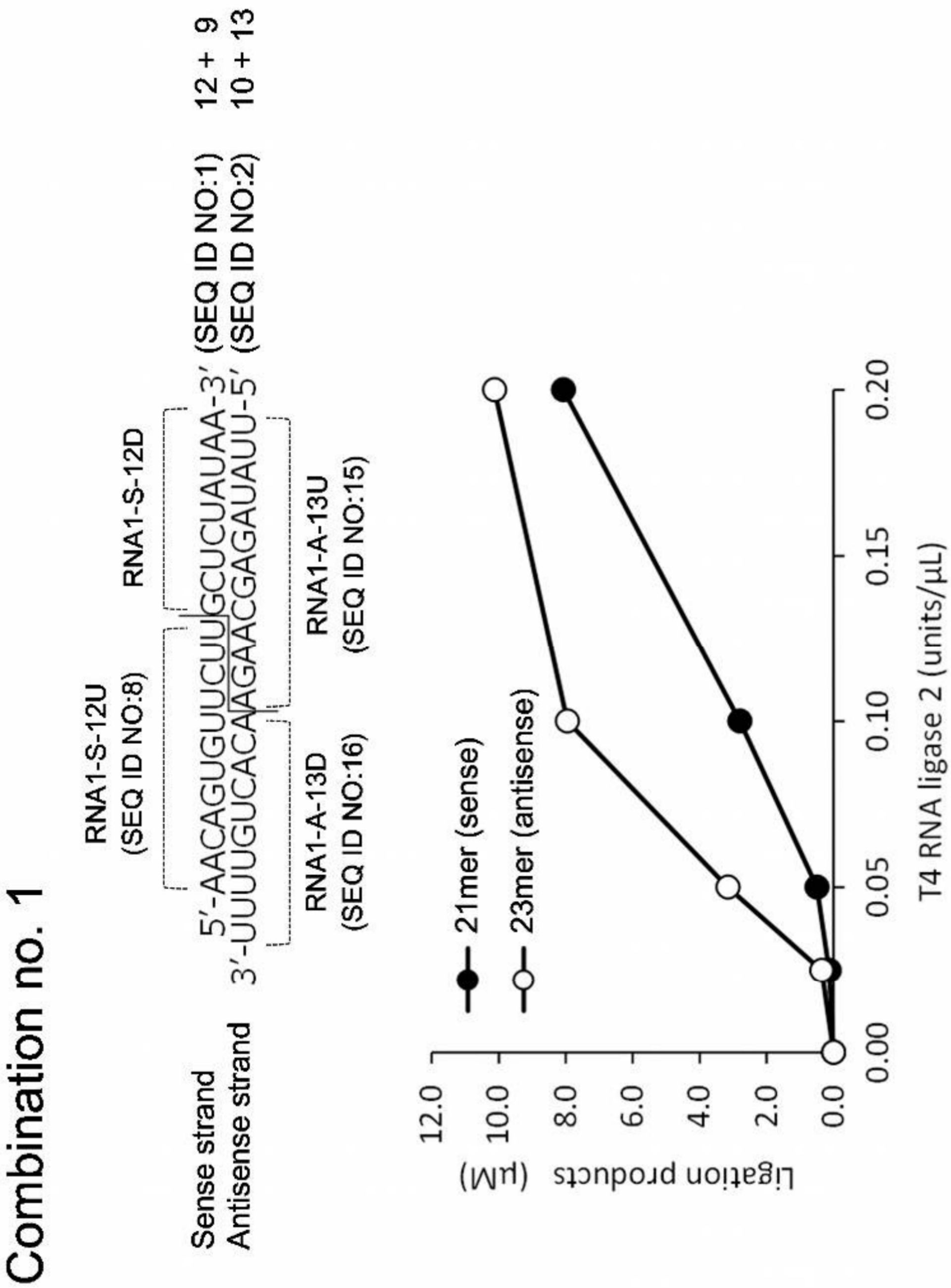
Combination no. 1
Sense strand
Antisense strand
RNA1-S-12U
(SEQ ID NO:8)
RNA1-S-12D
5′-AACAGUGUUCUUGCUCUAUAA-3′ (SEQ ID NO:1)
3′-UUUUGUCACAAGAACGAGAUAUU-5′ (SEQ ID NO:2)
12 + 9
10 + 13
RNA1-A-13D
(SEQ ID NO:16)
RNA1-A-13U
(SEQ ID NO:15)
21mer (sense)
23mer (antisense)
Ligation products (μM)
12.0
10.0
8.0
6.0
4.0
2.0
0.0
0.00
0.05
0.10
0.15
0.20
T4 RNA ligase 2 (units/μL)

FIG. 2-2

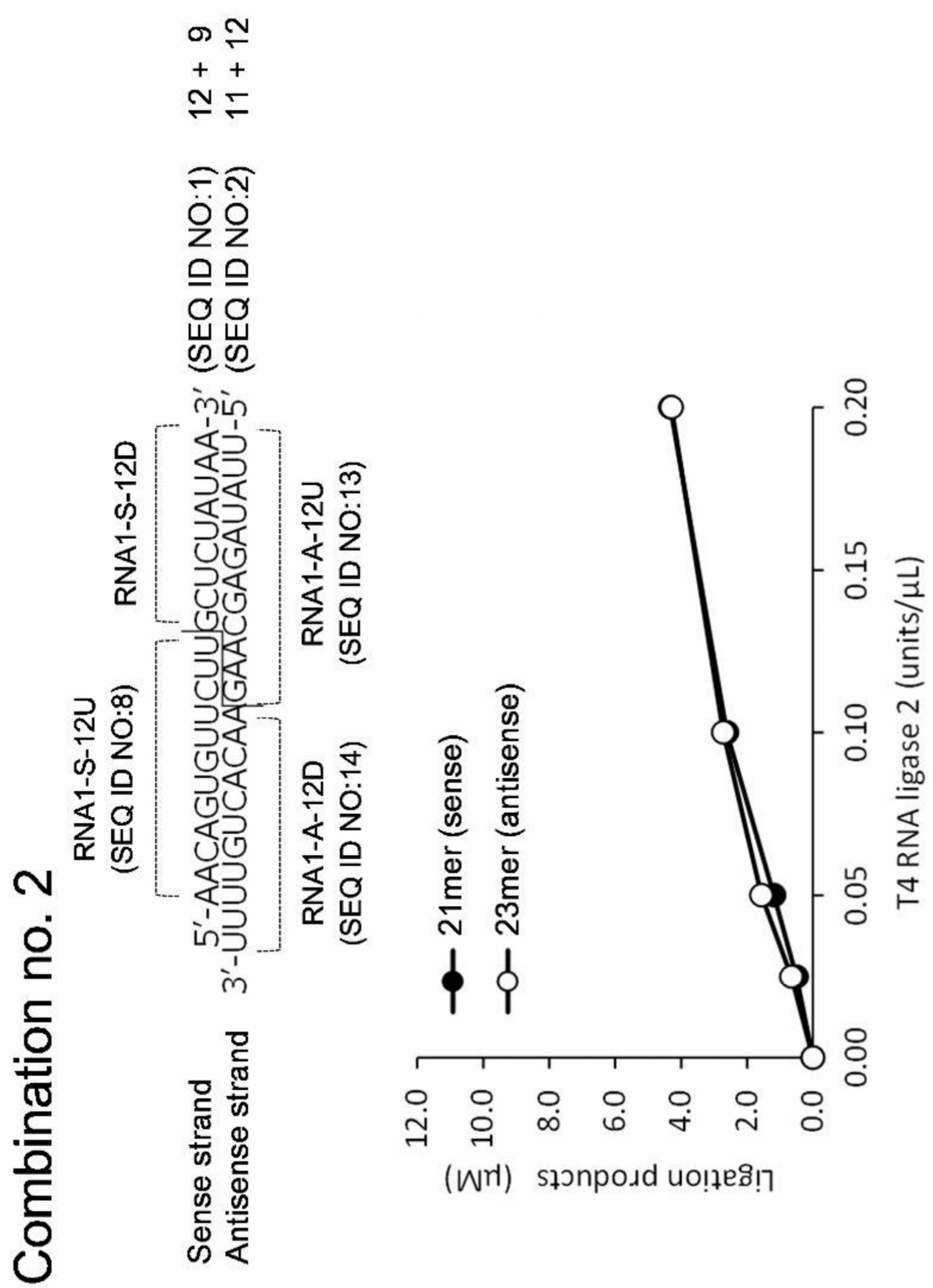
Combination no. 2
Sense strand
Antisense strand
RNA1-S-12U
(SEQ ID NO:8)
RNA1-S-12D
5'-AACAGUGUUCUUGCUCUAUAA-3'
3'-UUUUGUCACAAGAACGAGAUAUU-5'
(SEQ ID NO:1)
(SEQ ID NO:2)
12 + 9
11 + 12
RNA1-A-12D
(SEQ ID NO:14)
RNA1-A-12U
(SEQ ID NO:13)
21mer (sense)
23mer (antisense)
Ligation products (μM)
12.0
10.0
8.0
6.0
4.0
2.0
0.0
0.00
0.05
0.10
0.15
0.20
T4 RNA ligase 2 (units/μL)

# FIG. 2-3

## Combination no. 3

RNA1-S-12U (SEQ ID NO:8)    RNA1-S-12D

Sense strand 5′-AACAGUGUUCUUGCUCUAUAA-3′ (SEQ ID NO:1) 12 + 9
Antisense strand 3′-UUUUGUCACAAGAACGAGAUAUU-5′ (SEQ ID NO:2) 12 + 11

RNA1-A-11D (SEQ ID NO:12)    RNA1-A-11U (SEQ ID NO:11)

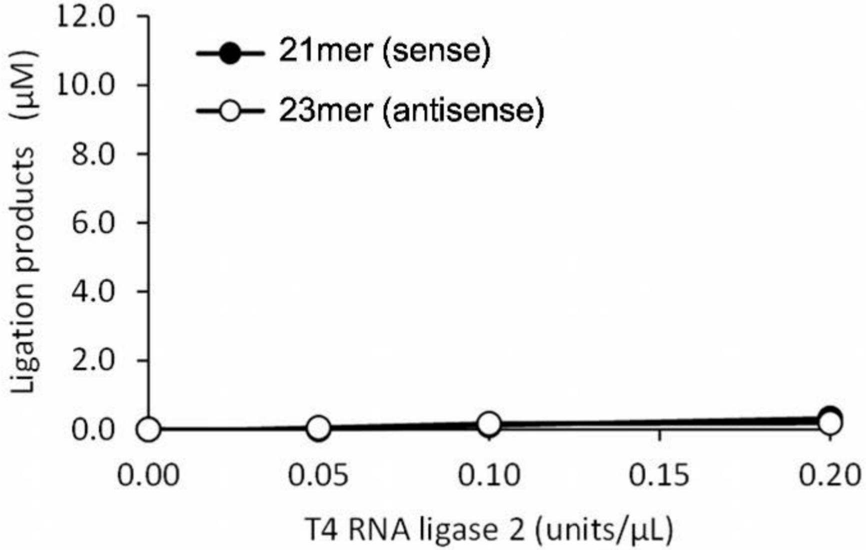

FIG. 2-4

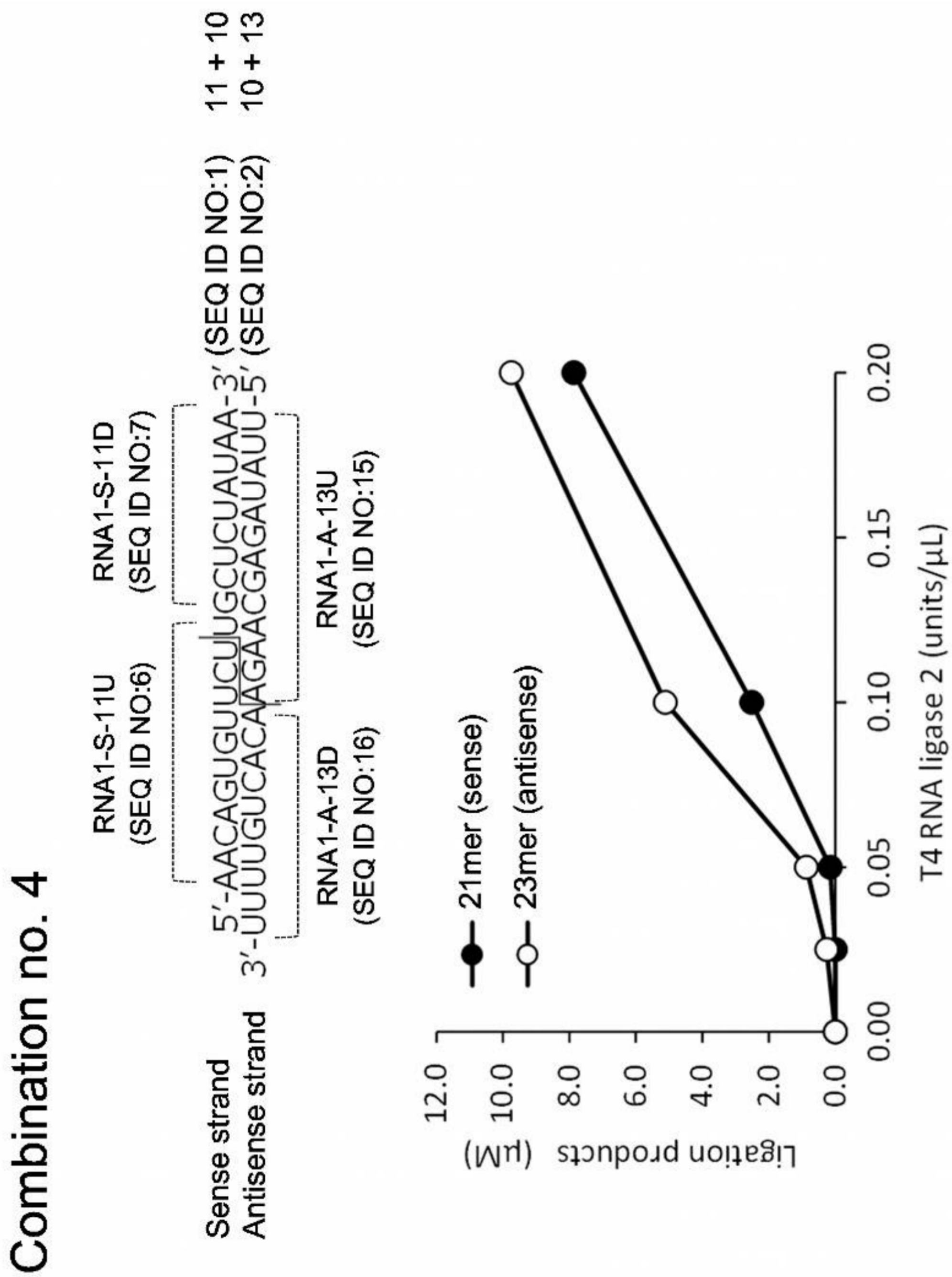
Combination no. 4
Sense strand
Antisense strand
RNA1-S-11U
(SEQ ID NO:6)
RNA1-S-11D
(SEQ ID NO:7)
5′-AACAGUGUUCUUGCUCUAUAA-3′ (SEQ ID NO:1)
3′-UUUUGUCACAAGAACGAGAUAUU-5′ (SEQ ID NO:2)
11 + 10
10 + 13
RNA1-A-13D
(SEQ ID NO:16)
RNA1-A-13U
(SEQ ID NO:15)
21mer (sense)
23mer (antisense)
Ligation products (μM)
12.0
10.0
8.0
6.0
4.0
2.0
0.0
0.00
0.05
0.10
0.15
0.20
T4 RNA ligase 2 (units/μL)

FIG. 2-5

Combination no. 5

RNA1-S-10U (SEQ ID NO:4) RNA1-S-10D (SEQ ID NO:5)

Sense strand 5′-AACAGUGUUCUUGCUCUAUAA-3′ (SEQ ID NO:1) 10 + 11

Antisense strand 3′-UUUUGUCACAAGAACGAGAUAUU-5′ (SEQ ID NO:2) 10 + 13

RNA1-A-13D (SEQ ID NO:16) RNA1-A-13U (SEQ ID NO:15)

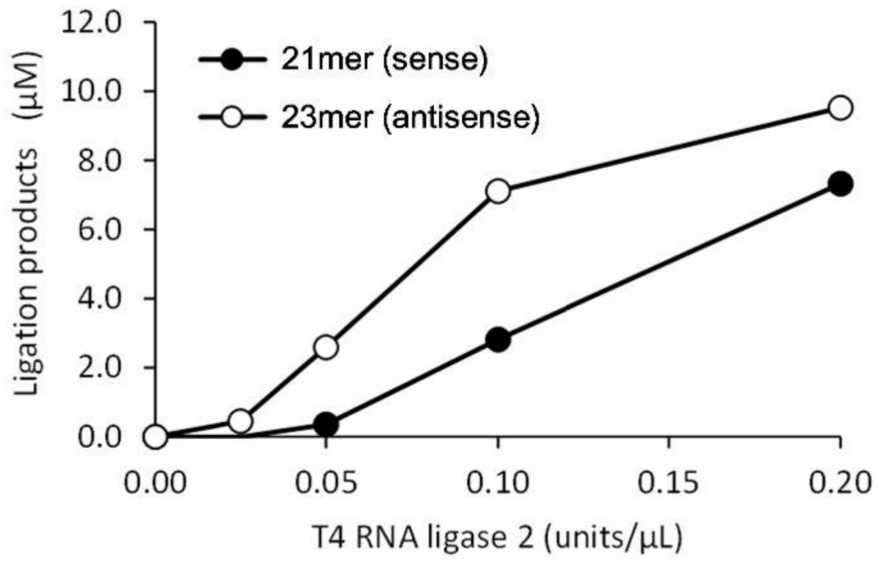

FIG. 2-6

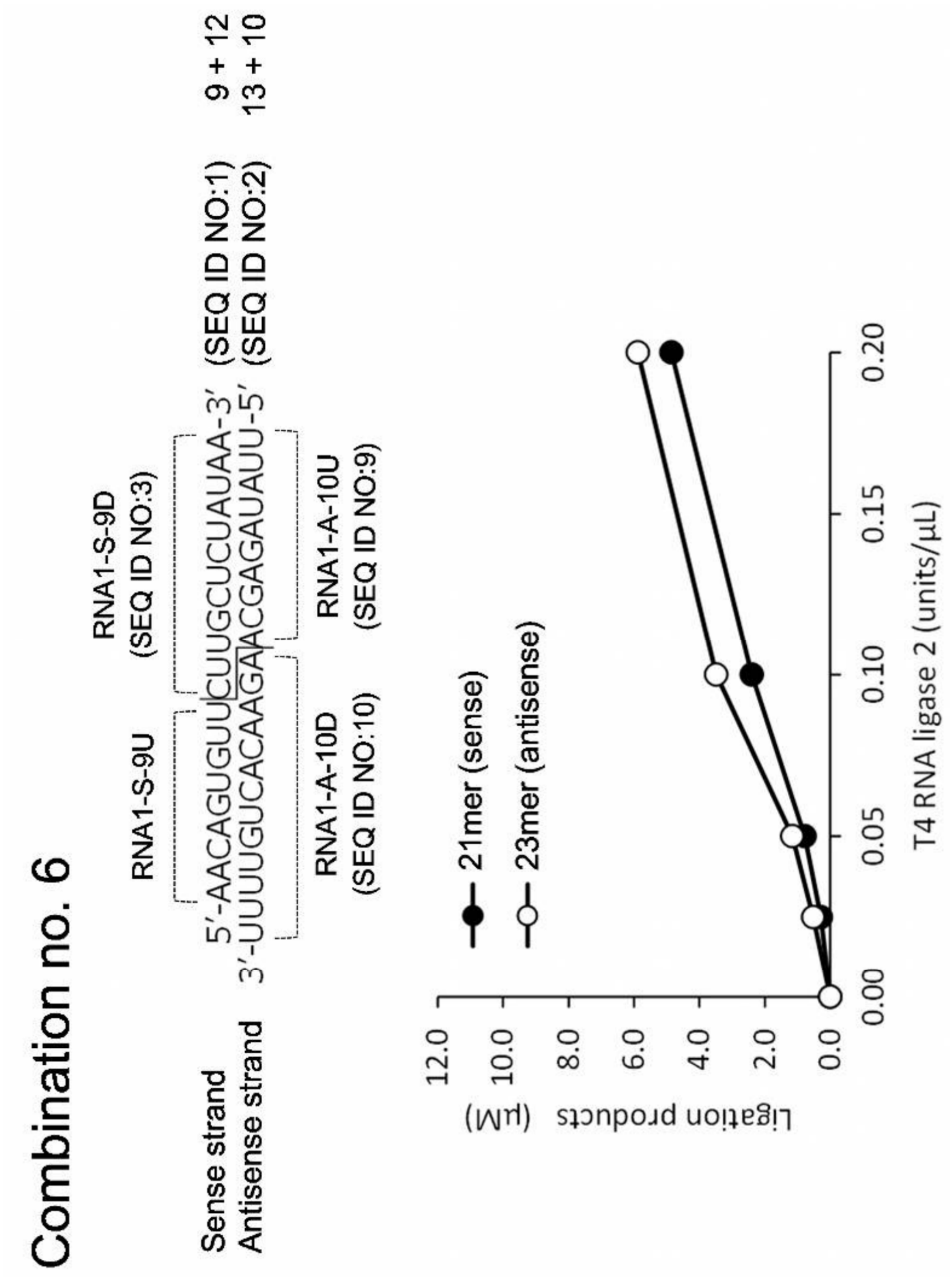
Combination no. 6
Sense strand
Antisense strand
RNA1-S-9U
RNA1-S-9D
(SEQ ID NO:3)
5'-AACAGUGUUCUUGCUCUAUAA-3' (SEQ ID NO:1) 9 + 12
3'-UUUUGUCACAAGAACGAGAUAUU-5' (SEQ ID NO:2) 13 + 10
RNA1-A-10D
(SEQ ID NO:10)
RNA1-A-10U
(SEQ ID NO:9)
21mer (sense)
23mer (antisense)
Ligation products (μM)
12.0
10.0
8.0
6.0
4.0
2.0
0.0
0.00
0.05
0.10
0.15
0.20
T4 RNA ligase 2 (units/μL)

FIG. 3-1

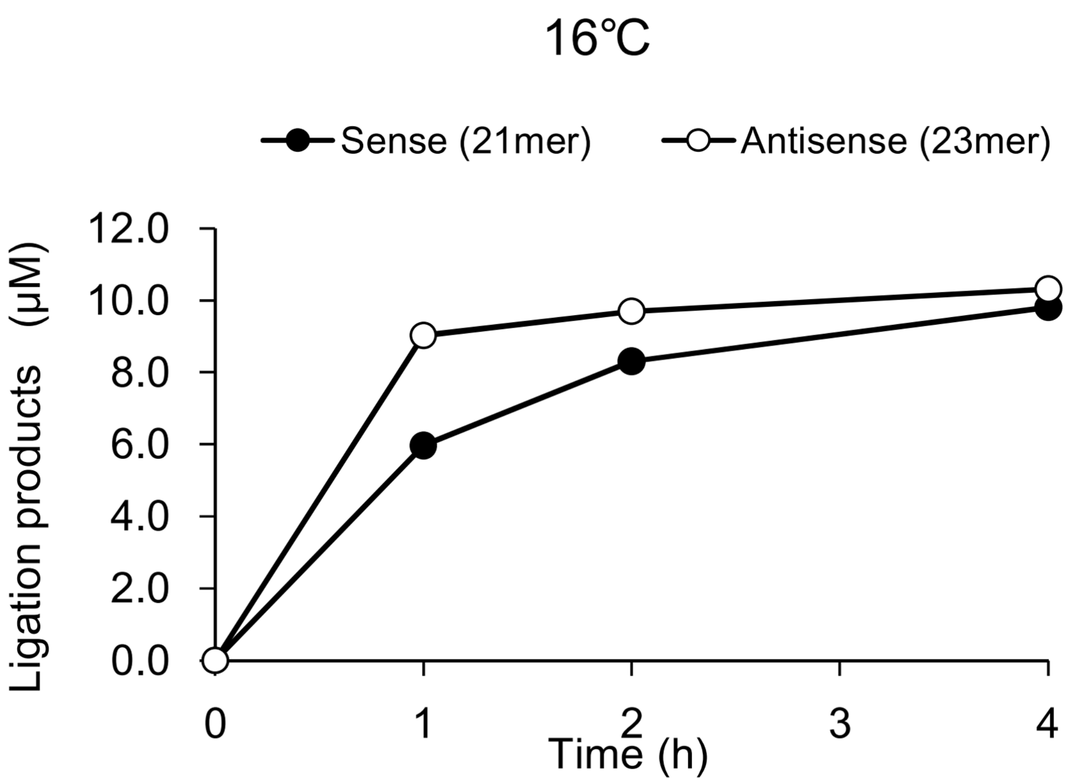
16℃
Sense (21mer)
Antisense (23mer)
Ligation products (μM)
12.0
10.0
8.0
6.0
4.0
2.0
0.0
0
1
2
3
4
Time (h)

FIG. 3-2

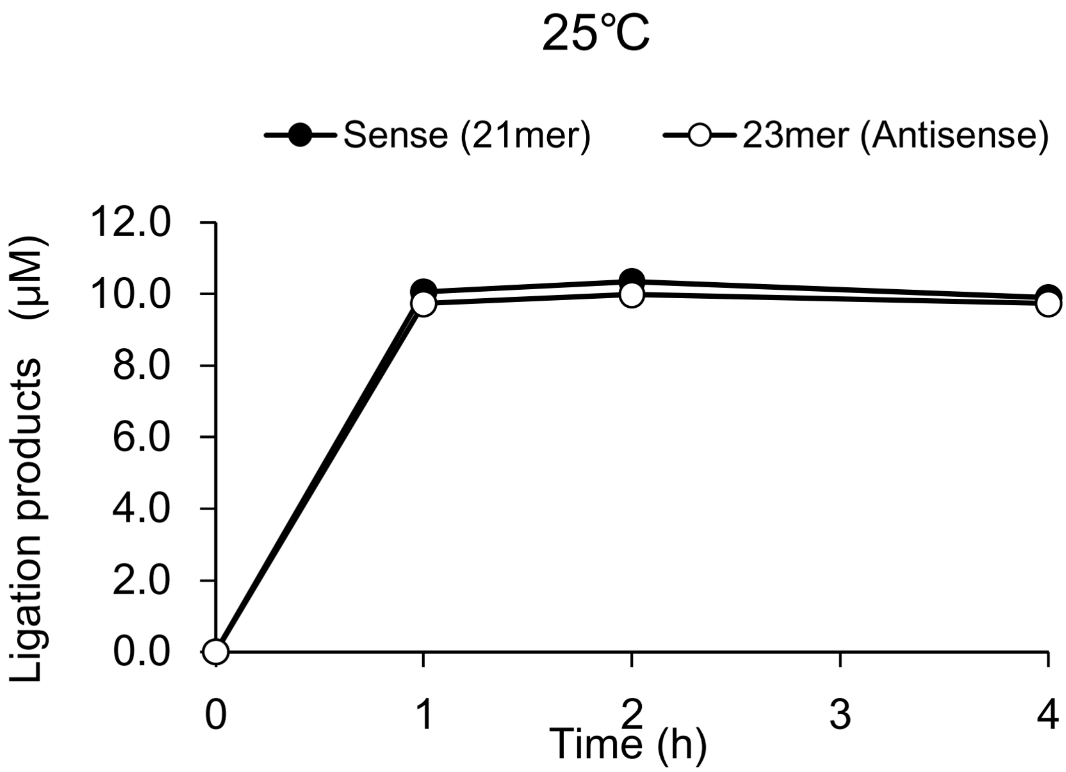
25°C
Sense (21mer)
23mer (Antisense)
Ligation products (μM)
12.0
10.0
8.0
6.0
4.0
2.0
0.0
0
1
2
3
4
Time (h)

FIG. 3-3

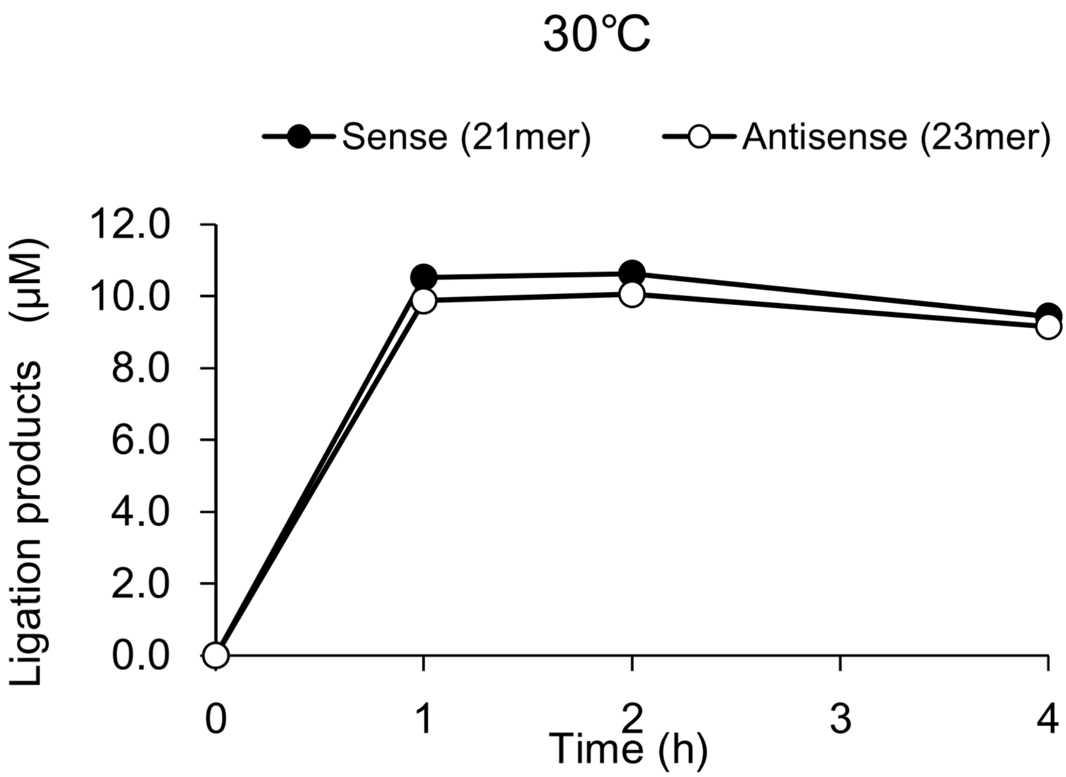
30℃
Sense (21mer)
Antisense (23mer)
Ligation products (μM)
12.0
10.0
8.0
6.0
4.0
2.0
0.0
0
1
2
3
4
Time (h)

FIG. 3-4

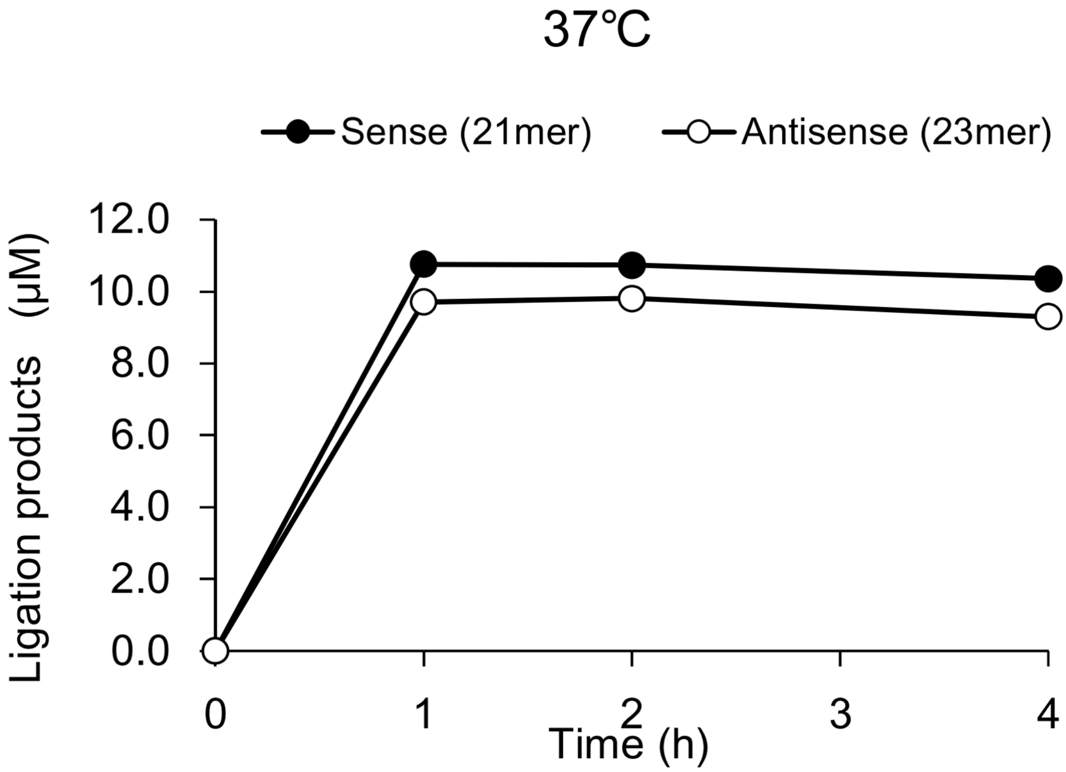
37℃
Sense (21mer)
Antisense (23mer)
Ligation products (μM)
12.0
10.0
8.0
6.0
4.0
2.0
0.0
0
1
2
3
4
Time (h)

FIG. 4

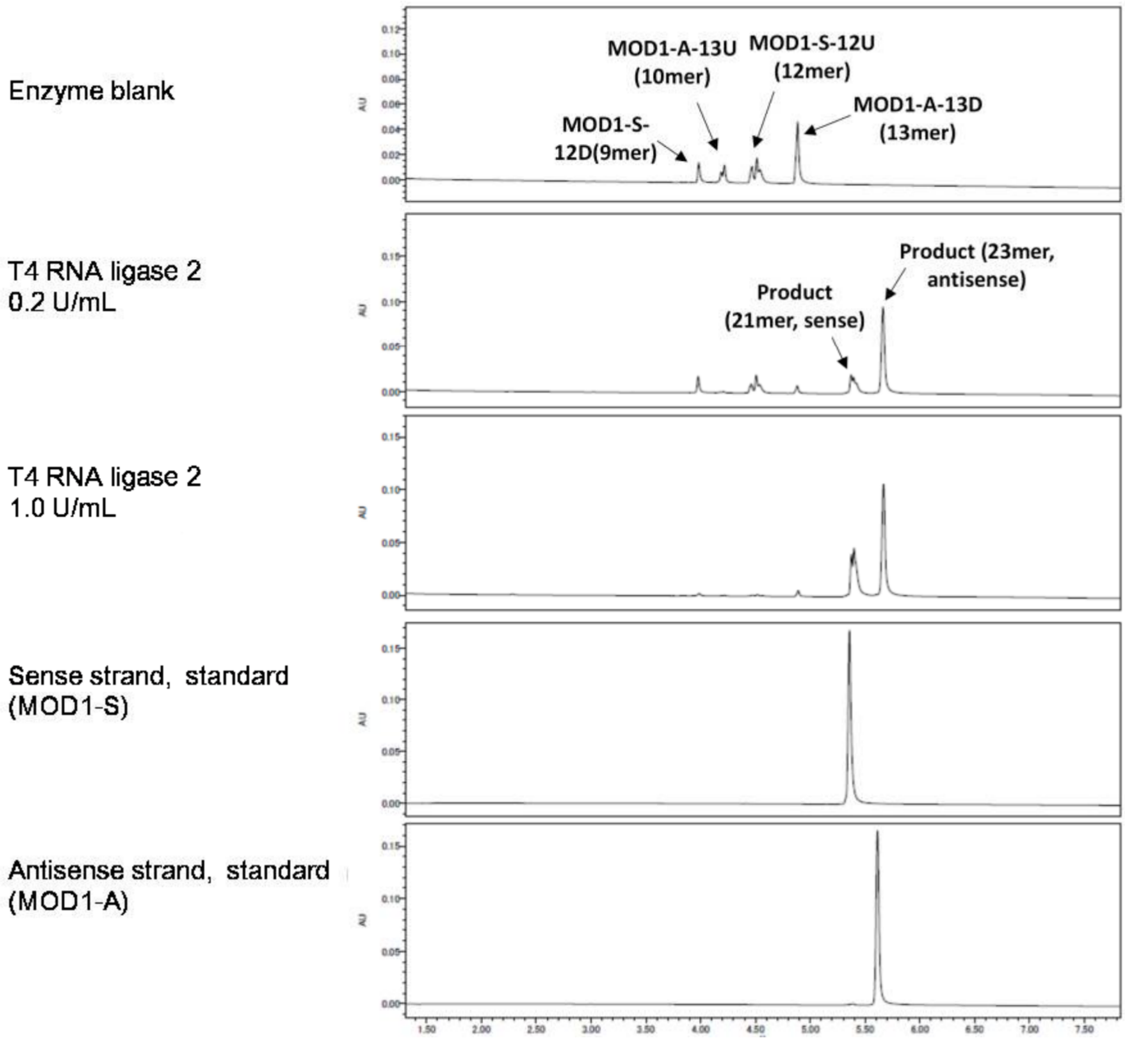
Enzyme blank
MOD1-A-13U (10mer)
MOD1-S-12U (12mer)
MOD1-A-13D (13mer)
MOD1-S-12D(9mer)
T4 RNA ligase 2 0.2 U/mL
Product (23mer, antisense)
Product (21mer, sense)
T4 RNA ligase 2 1.0 U/mL
Sense strand, standard (MOD1-S)
Antisense strand, standard (MOD1-A)
AU

FIG. 5

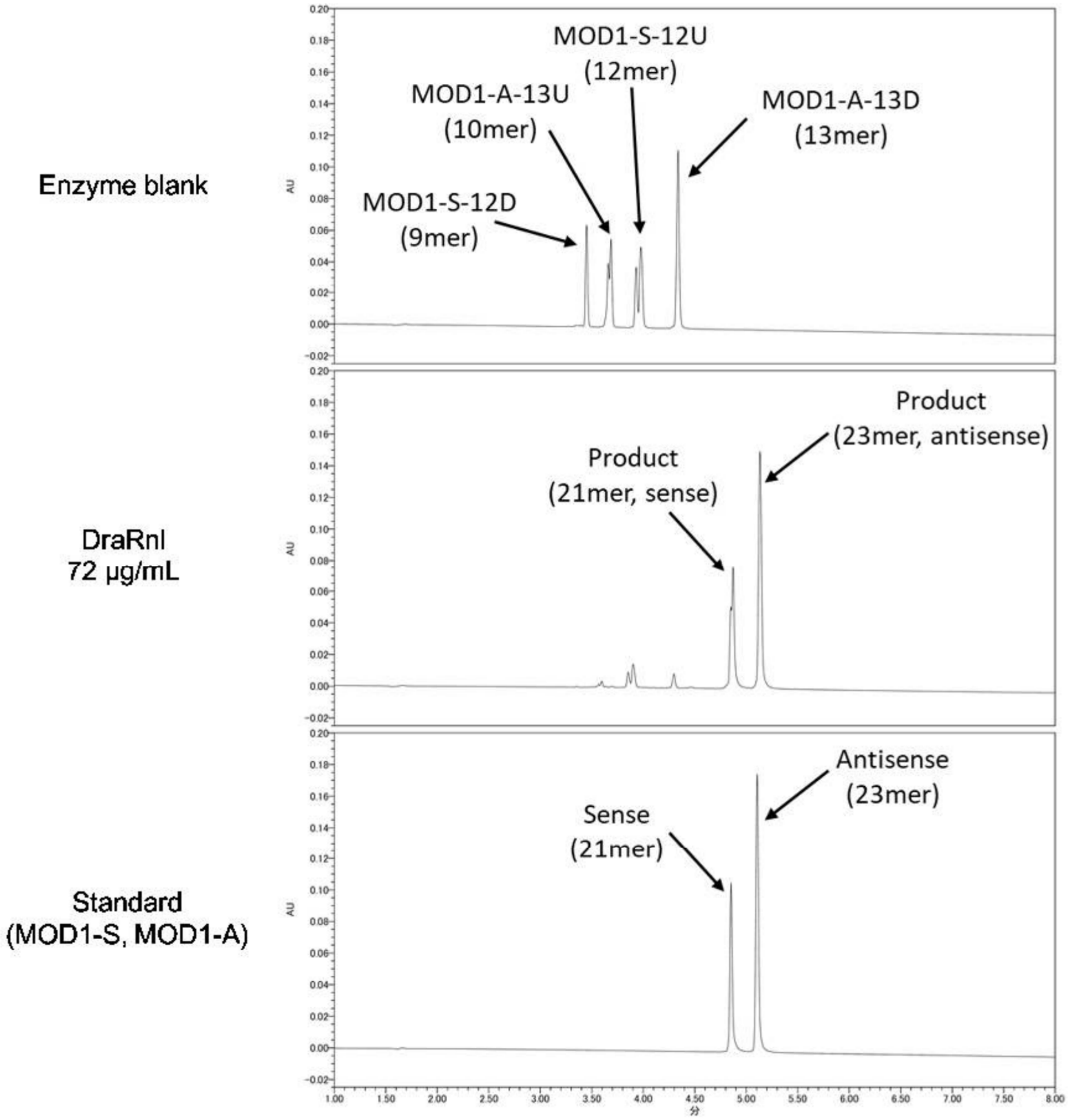
Enzyme blank
MOD1-S-12U
(12mer)
MOD1-A-13U
(10mer)
MOD1-A-13D
(13mer)
MOD1-S-12D
(9mer)
DraRnl
72 μg/mL
Product
(23mer, antisense)
Product
(21mer, sense)
Standard
(MOD1-S, MOD1-A)
Antisense
(23mer)
Sense
(21mer)
AU
分

# FIG. 6

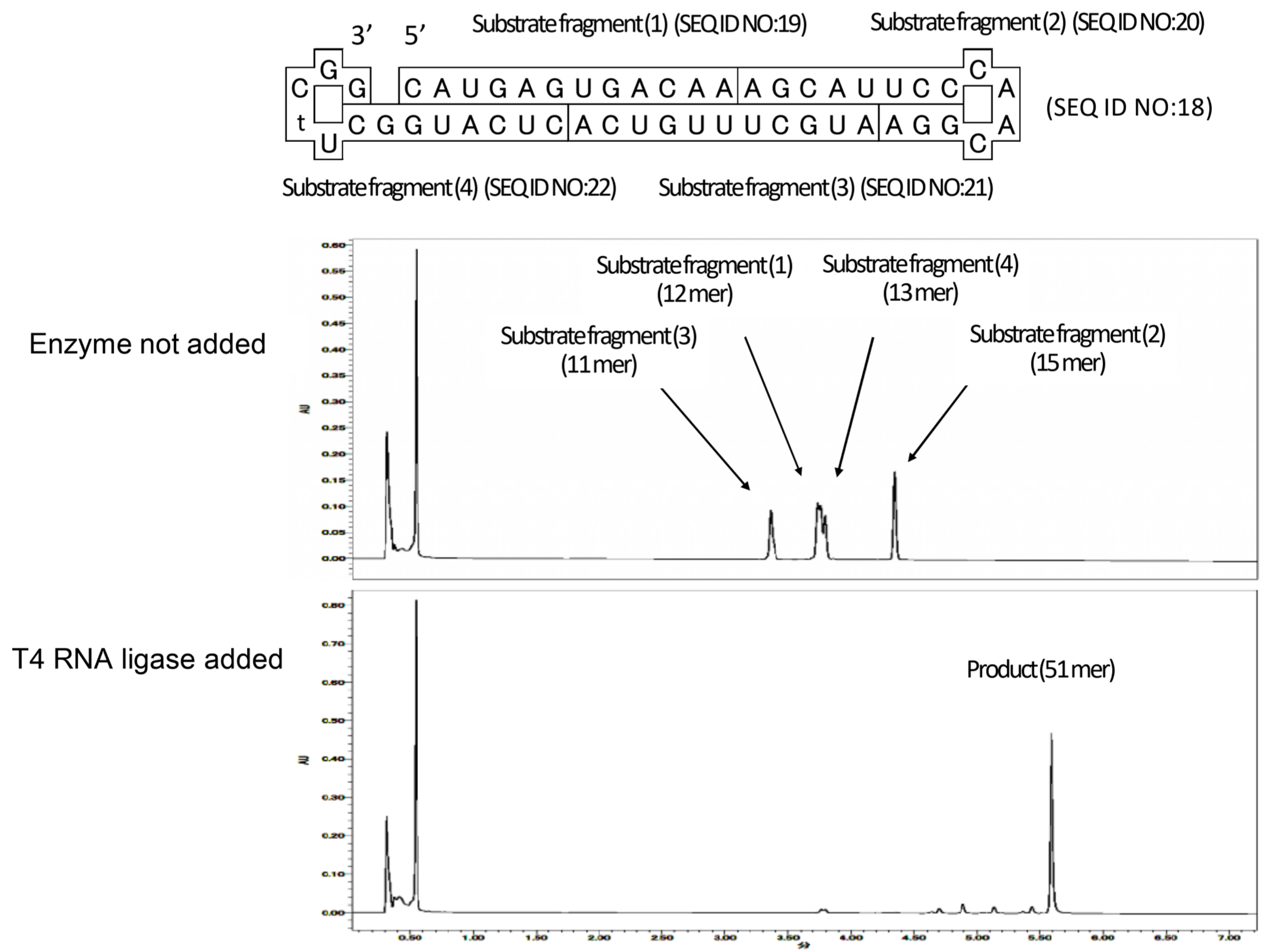
3'
5'
Substrate fragment (1) (SEQ ID NO:19)
Substrate fragment (2) (SEQ ID NO:20)
C A U G A G U G A C A A A G C A U U C C
C G G U A C U C A C U G U U U C G U A A G G
(SEQ ID NO:18)
Substrate fragment (4) (SEQ ID NO:22)
Substrate fragment (3) (SEQ ID NO:21)
Enzyme not added
Substrate fragment (3) (11 mer)
Substrate fragment (1) (12 mer)
Substrate fragment (4) (13 mer)
Substrate fragment (2) (15 mer)
T4 RNA ligase added
Product (51 mer)

# FIG. 7

Mismatch
↓

Strand A 5′ AF^ Am^ CF Gm GF Um GF Um UF CF UF Um GF Cm UF Cm UF Am UF^ Am^ AF 3′

Strand B 3′ Um^Um^ Um UF Gm UF Cm AF Cm AF Am Gm Am AF Cm GF Am GF Am UF Am UF Um 5′

Nucleotide sequence of Strand A : SEQ ID NO: 25
Nucleotide sequence of Strand B : SEQ ID NO: 26
English capital letter: RNA
m: modification by 2′-O-methyl
F: 2′-fluoro group
^: thiophosphate linkage
dT: thymidine

# FIG. 8

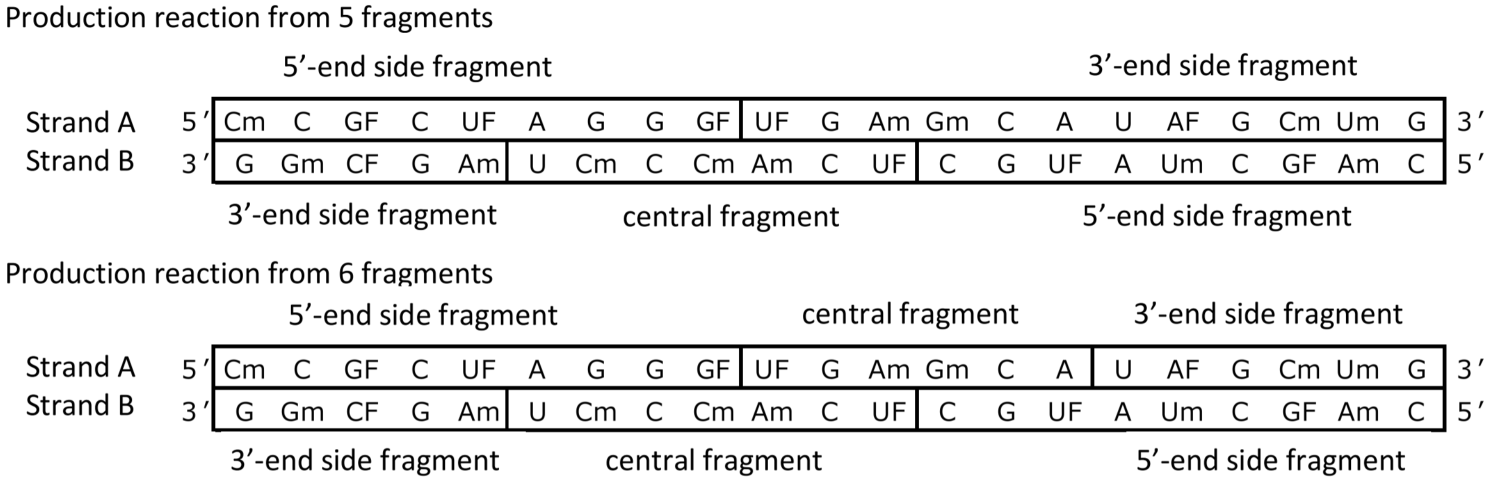


Production reaction from 5 fragments
Nucleotide sequence of Strand A : SEQ ID NO: 30
Nucleotide sequence of Strand B : SEQ ID NO: 31

Production reaction from 6 fragments
Nucleotide sequence of Strand A : SEQ ID NO: 30
Nucleotide sequence of Strand B : SEQ ID NO: 31

English capital letter: RNA
m: modification by 2'-O-methyl group

FIG. 9

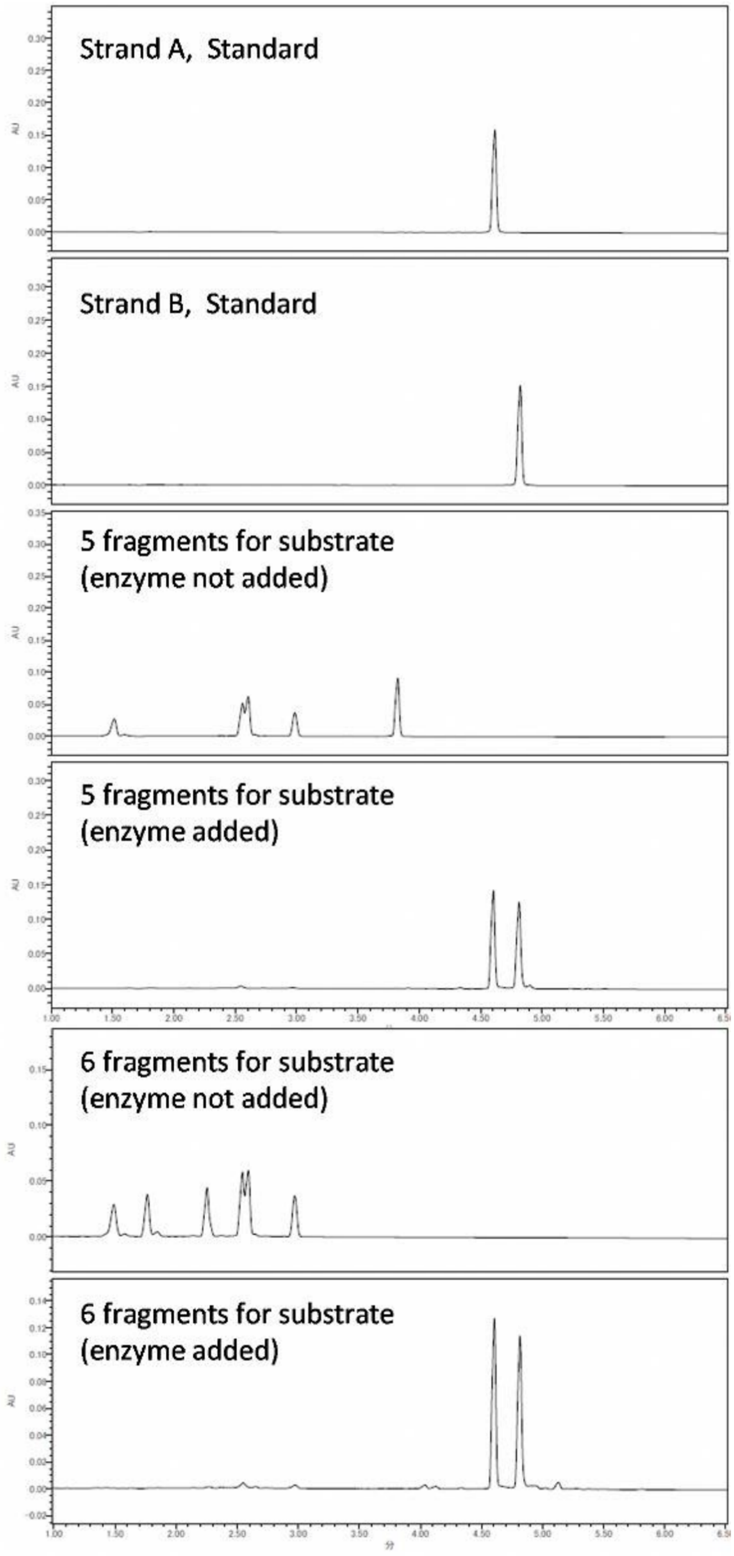
Strand A, Standard
Strand B, Standard
5 fragments for substrate
(enzyme not added)
5 fragments for substrate
(enzyme added)
6 fragments for substrate
(enzyme not added)
6 fragments for substrate
(enzyme added)

# FIG. 10

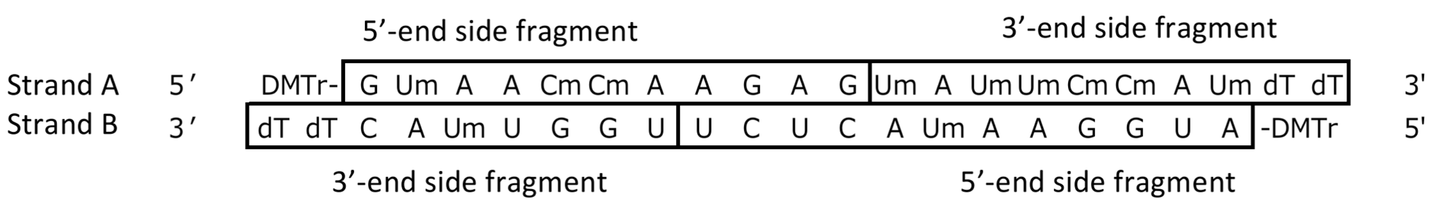


Nucleotide sequence of Strand A : SEQ ID NO: 33
Nucleotide sequence of Strand B : SEQ ID NO: 34
English capital letter: RNA
m: modification by 2'-O-methyl group
^: thiophosphate linkage
dT: thymidine
DMTr: modification at 5'-end by DMTr group

# FIG. 11

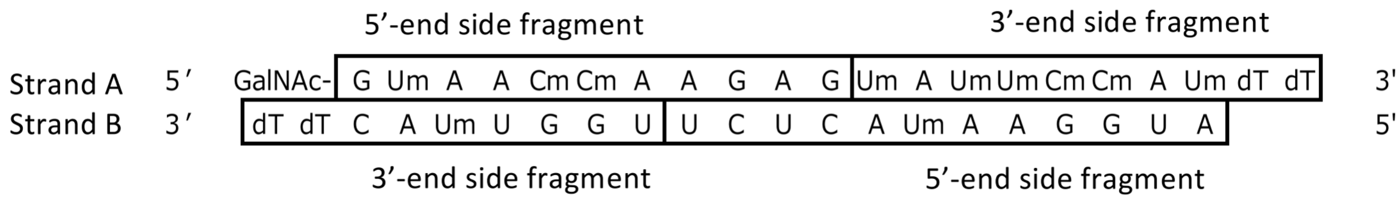


Nucleotide sequence of Strand A : SEQ ID NO: 33
Nucleotide sequence of Strand B : SEQ ID NO: 34
English capital letter: RNA
m: modification by 2’-O-methyl group
dT: thymidine
5’-end modified by addition of carrier

# FIG. 12

5’-end side fragment | 3’-end side fragment

Strand A 5′ G^ Um^ A^ A^ Cm^Cm^ A^ A^ G^ A^ G^ | Um^ A^ Um^Um^Cm^Cm^ A^ Um^ dT^ dT 3'

Strand B 3′ dT^ dT^ C^ A^ Um^ U^ G^ G^ U^ | U^ C^ U^ C^ A^ Um^ A^ A^ G^ G^ U^ A 5'

3’-end side fragment | 5’-end side fragment

Nucleotide sequence of Strand A : SEQ ID NO: 33
Nucleotide sequence of Strand B : SEQ ID NO: 34
English capital letter: RNA
m: modification by 2’-O-methyl group
^: thiophosphate linkage
dT: thymidine

FIG. 13

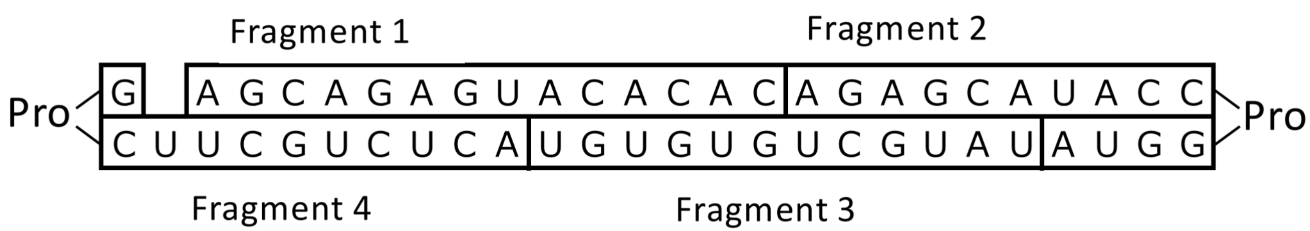


Nucleotide sequence of linking product of fragments 1, 2, 3 and 4 (SEQ ID NOs:39-42)
English capital letter: RNA
Pro: proline derivative

# FIG. 14

Reaction of sticky end (length: 6 nt)

5'-end side fragment | 3'-end side fragment

Strand A 5' G Cm Um A A Cm Cm G G G G G G | G Um G Cm Cm A Gm 3'
Strand B 3' Cm G A Um U G G Cm | Cm Cm Cm Cm Cm Cm Cm A Cm G G U C 5'

3'-end side fragment | 5'-end side fragment

Reaction of sticky end (length: 5 nt)

5'-end side fragment | 3'-end side fragment

Strand A 5' G Cm Um A A Cm Cm G G G G G | G G Um G Cm Cm A Gm 3'
Strand B 3' Cm G A Um U G G Cm | Cm Cm Cm Cm Cm Cm Cm A Cm G G U C 5'

3'-end side fragment | 5'-end side fragment

Reaction of sticky end (length: 4 nt)

5'-end side fragment | 3'-end side fragment

Strand A 5' G Cm Um A A Cm Cm G G G G | G G G Um G Cm Cm A Gm 3'
Strand B 3' Cm G A Um U G G Cm | Cm Cm Cm Cm Cm Cm Cm A Cm G G U C 5'

3'-end side fragment | 5'-end side fragment

Reaction of sticky end (length: 3 nt)

5'-end side fragment | 3'-end side fragment

Strand A 5' G Cm Um A A Cm Cm G G G | G G G G Um G Cm Cm A Gm 3'
Strand B 3' Cm G A Um U G G Cm | Cm Cm Cm Cm Cm Cm Cm A Cm G G U C 5'

3'-end side fragment | 5'-end side fragment

Reaction of sticky end (length: 2 nt)

5'-end side fragment | 3'-end side fragment

Strand A 5' G Cm Um A A Cm Cm G G | G G G G G Um G Cm Cm A Gm 3'
Strand B 3' Cm G A Um U G G Cm | Cm Cm Cm Cm Cm Cm Cm A Cm G G U C 5'

3'-end side fragment | 5'-end side fragment

Reaction of sticky end (length: 1 nt)

5'-end side fragment | 3'-end side fragment

Strand A 5' G Cm Um A A Cm Cm G | G G G G G G Um G Cm Cm A Gm 3'
Strand B 3' Cm G A Um U G G Cm | Cm Cm Cm Cm Cm Cm Cm A Cm G G U C 5'

3'-end side fragment | 5'-end side fragment

Nucleotide sequence of Strand A as shown above : SEQ ID NO: 43
Nucleotide sequence of Strand B as shown above : SEQ ID NO: 44
English capital letter: RNA
m: modification by 2'-O-methyl group

# FIG. 15

(1) Strand length of product: 14 oligonucleotides

(a) Strand A 5′ Gm C Cm Cm A Cm G A | Um A G Um G Cm 3′
(b) Strand B 3′ C Gm G G Um | G C Um A Um Cm A Cm G 5′

(2) Strand length of product: 13 oligonucleotides

(c) Strand A 5′ Gm C Cm Cm A Cm G A | Um G Um G Cm 3′
(d) Strand B 3′ C Gm G G Um | G C Um A Cm A Cm G 5′

(3) Strand length of product: 12 oligonucleotides

(e) Strand A 5′ Gm C Cm Cm A C G | Um G Um G Cm 3′
(f) Strand B 3′ C Gm G G Um | G C A Cm A Cm G 5′

(4) Strand length of product: 11 oligonucleotides

(g) Strand A 5′ Gm C Cm Cm A C G | Um G Um G 3′
(h) Strand B 3′ C Gm G G Um | G C A Cm A Cm 5′

Nucleotide sequence of (a) - (h) : SEQ ID NOs: 54-61
English capital letter: RNA
m: modification by 2'-O-methyl group

# FIG. 16

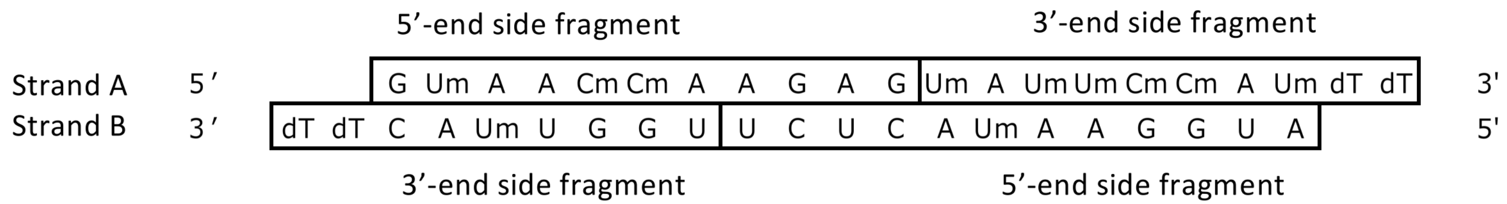


Nucleotide sequence of Strand A : SEQ ID NO: 33
Nucleotide sequence of Strand B : SEQ ID NO: 34
English capital letter: RNA
m: modification by 2’-O-methyl group
dT: thymidine

# FIG. 17

| | | | |
|---|---|---|---|
| DNA (28 nt) | (a) Strand A | 5' | g g g t a a t g a a t t c c a a a g c t t c c c c c t a 3' |
| | (b) Strand B | 3' | c c c a t t a c t t a a g g t t t c g a a g g g g g a t 5' |
| Modified oligonucleotide (28 nt) | (c) Strand A | 5' | Gm G Gm U A A U Gm A A Um U Cm C A Am Am G C U U Cm C C C Cm U Am 3' |
| | (d) Strand B | 3' | C Cm C A Um U Am C U U A A G Gm U U U C Gm A Am G G Gm G G Am U 5' |
| Modified oligonucleotide (25 nt) | (e) Strand A | 5' | Gm G Gm U A A U Gm A A Um U Cm C A Am Am G C U U Cm C C C 3' |
| | (f) Strand B | 3' | C Cm C A Um U Am C U U A A G Gm U U U C Gm A Am G G Gm G 5' |
| Modified oligonucleotide (23 nt) | (g) Strand A | 5' | Gm G Gm U A A U Gm A A Um U Cm C A Am Am G C U U Cm C 3' |
| | (h) Strand B | 3' | C Cm C A Um U Am C U U A A G Gm U U U C Gm A Am G G 5' |
| Modified oligonucleotide (21 nt) | (i) Strand A | 5' | Gm G Gm U A A U Gm A A Um U Cm C A Am Am G C U U 3' |
| | (j) Strand B | 3' | C Cm C A Um U Am C U U A A G Gm U U U C Gm A Am 5' |

Nucleotide sequence of (a) - (j) : SEQ ID NOs: 62, 63, 67, 68, 72, 73, 76, 77, 80 and 81
English small letter: DNA
English capital letter: RNA
m: modification by 2'-O-methyl group

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20180023122 A
- WO 2012005368 A
- WO 2012157723 A
- WO 2005070859 A


### Non-patent literature cited in the description


- **SOSIC A** ; **PASQUALIN M** ; **PASUT G** ; **GATTO B**. Enzymatic formation of PEGylated oligonucleotides. *Bioconjug Chem*, 2014, vol. 25, 433-441
- **BULLARD, D. R.** ; **BOWATER R. P.** Direct comparison of nick-joining activity of the nucleic acid ligases from bacteriophage T4. *Biochem. J*, 2006, vol. 398, 135-144
- **NANDAKUMAR, J.** ; **SHUMAN, S.** How an RNA Ligase Discriminates RNA versus DNA Damage. *Molecular Cell*, 2004, vol. 16 (2), 211-221
- **NANDAKUMAR, J** ; **HO CK** ; **LIMA CD** ; **SHUMAN S**. RNA substrate specificity and structure-guided mutational analysis of bacteriophage T4 RNA ligase 2. *J Biol Chem*, 2004, vol. 279, 31337-31347
- **JAYAPRAKASH K. NAIR et al.** Multivalent N-Acetylgalactosamine-Conjugated siRNA Localizes in Hepatocytes and Elicits Robust RNAi-Mediated Gene Silencing. *J. Am. Chem. Soc.*, 2014, vol. 136, 16958-16961
- **MIHAELA-CARMEN UNCIULEAC** ; **STEWART SHUMAN**. *RNA*, 2019, vol. 21, 824-832
- **HAMASAKI T.** ; **SUZUKI H.** ; **SHIROHZU H. et al.** Efficacy of a novel class of RNA interference therapeutic agents. *PLoS ONE*, 2012, vol. 7 (8), e42655
- **ROUSSIS et al.** *Journal of Chromatogr A.*, 2019, vol. 1584, 106-114